# EUROPEAN PATENT APPLICATION

(11) **EP 4 570 852 A1**
(43) Date of publication of application: **18.06.2025**
(21) Application number: 23852571.1
(22) Date of filing: 08.08.2023
(51) Int. Cl.: C08J 11/22, C07C 67/03, C08G 63/78, C07C 69/82

(54) **POLYESTER DECOMPOSITION METHOD, POLYESTER PRODUCTION METHOD, AND POLYESTER DECOMPOSITION PRODUCT RECOVERY METHOD**

(30) Priority: 09.08.2022 JP 2022126948; 09.08.2022 JP 2022126949
(71) Applicant: National Institute of Advanced Industrial Science and Technology, Chiyoda-ku Tokyo 100-8921 (JP)
(72) Inventor: TANAKA Shinji, Tsukuba-shi, Ibaraki 305-8560 (JP); NAKAJIMA Yumiko, Tsukuba-shi, Ibaraki 305-8560 (JP); SATO Kazuhiko, Tsukuba-shi, Ibaraki 305-8560 (JP); KURAGANO Takashi, Tsukuba-shi, Ibaraki 305-8560 (JP); OGAWA Atsuko, Tsukuba-shi, Ibaraki 305-8560 (JP)
(74) Representative: van Dam, Vincent
(86) International application number: PCT/JP2023/028929
(87) International publication number: WO 2024/034609

(57) **Abstract**

Provided are: a polyester decomposition method capable of decomposing a polyester or polyester contained in various polyester-containing materials into monomers at a low temperature of 150°C or lower in a simple process; a polyester production method for producing a polyester from the monomers obtained by the decomposition method; and a polyester decomposition product recovery method for recovering a predetermined polyester decomposition product from polyester decomposition products obtained by the polyester decomposition method.

The polyester decomposition method of a polyester contained in a polyester-containing material includes a decomposition step of mixing a base, a monohydric alcohol, and a carbonic acid diester to decompose the polyester.

## Description

### Technical Field

The present invention relates to: a polyester decomposition method for decomposing a polyester; a polyester production method for producing a polyester from monomers obtained by this polyester decomposition method; and a polyester decomposition product recovery method for recovering a predetermined polyester decomposition product from polyester decomposition products obtained by this polyester decomposition method.

### Background Art

In recent years, the development of plastic recycling technology has been strongly encouraged due to concerns about environmental destruction, including marine pollution. Polyesters are widely used as a material of bottles and fibers, and the global annual production of polyethylene terephthalate (PET), in particular, reaches 80 million tons. In the polyester recycling technology, a material recycling method that does not involve depolymerization and a chemical recycling method that involves depolymerization and repolymerization have been developed. The former method is easily applied to polyesters used for PET bottles or the like due to the high purity, but hardly applied to materials containing polyesters (polyester-containing materials) such as polyester fibers and a film containing a polyester.

As the method for depolymerizing a polyester, methods using water and a supercritical alcohol are known (see Patent Literatures 1 and 2). However, the methods both require a high temperature condition of 300°C or higher. On the other hand, a transesterification method using a base catalyst and an alcohol can achieve depolymerization at a relatively low temperature. A method using methanol can achieve depolymerization at a low temperature of about room temperature (15 to 25°C) to 50°C by using a halogenated solvent as well as a potassium carbonate (see NPL 1) or an alkali metal alkoxide (see PTL 3). Another known method is using dimethyl carbonate as an ethylene glycol scavenger to make depolymerization more efficient (Non-Patent Literature 2). However, an object to be depolymerized by these methods is limited to high-purity PET derived from PET bottles or the like, and these methods are not applicable to other polyester-containing materials. A method of excessively using a base catalyst and an ethylene glycol is known as a depolymerization method of colored polyester fibers. However, this method requires a high temperature of about 200°C as well as decoloring of fibers with a high boiling point solvent (see Patent Literatures 4 to 6) or decomposition of a dye with an oxidant (see Patent Literature 7) to obtain high-purity monomers.

### Citation List

### Patent Literature

PTL 1: Japanese Patent No. 5099416
PTL 2: Japanese Patent Application Laid-Open No. 2001-39908
PTL 3: US Patent No. 10252976
PTL 4: Japanese Patent No. 4537288
PTL 5: Japanese Patent No. 5134563
PTL 6: Japanese Patent No. 6659919
PTL 7: Japanese Patent No. 6986813

### Non Patent Literature

NPL 1: Green Chem. 2021, 23, 511.
NPL 2: Green Chem. 2021, 23, 9412.

### Summary of Invention

### Technical Problem

However, there has been a problem that a polyester or polyester contained in various polyester-containing materials cannot be decomposed into monomers at a low temperature of 150°C or lower in a simple process.

An object of the present invention is to provide: a polyester decomposition method capable of decomposing a polyester or polyester contained in various polyester-containing materials into monomers at a low temperature of 150°C or lower in a simple process; a polyester production method for producing a polyester from the monomers obtained by the polyester decomposition method; and a polyester decomposition product recovery method for recovering a predetermined polyester decomposition product from polyester decomposition products obtained by the polyester decomposition method.

### Solution to Problem

The inventors of the present invention conducted extensive studies to address the above-described problems, and as a result, have found a breakthrough polyester decomposition method, polyester production method, and polyester decomposition product recovery method described below.

A first aspect of the present invention for solving the above-described problems is a polyester decomposition method of a polyester contained in a polyester-containing material, the method including a decomposition step of mixing a base, a monohydric alcohol, and a carbonic acid diester to decompose the polyester.

Here, the "decomposition" is a concept encompassing depolymerization.

According to the first aspect, the polyester contained in the polyester-containing material can be decomposed into monomers in a simple process.

A second aspect of the present invention is the polyester decomposition method according to the first aspect, in which a reaction temperature in the decomposition step is in a range of 20°C to 150°C.

According to the second aspect, the polyester contained in the polyester-containing material can be decomposed into monomers at a low temperature of 150°C or lower in a simple process.

A third aspect of the present invention is the polyester decomposition method according to the first or second aspect, in which the base is an alkali metal carbonate, an alkali metal hydroxide, or an alkali metal alkoxide.

According to the third aspect, the polyester contained in the polyester-containing material can be decomposed into monomers with higher purity.

A fourth aspect of the present invention is the polyester decomposition method according to the first or second embodiment, in which the monohydric alcohol is methanol.

According to the fourth aspect, the polyester contained in the polyester-containing material can be decomposed into monomers with higher purity.

A fifth aspect of the present invention is the polyester decomposition method according to the first or second aspect, in which the carbonic acid diester is dimethyl carbonate.

According to the fifth aspect, the polyester contained in the polyester-containing material can be decomposed into monomers with higher purity.

A sixth aspect of the present invention is the polyester decomposition method according to the first or second aspect, in which the decomposition step includes mixing the base, the monohydric alcohol, and the carbonic acid diester using a mechanochemical reaction device to decompose the polyester.

According to the sixth aspect, the polyester contained in the polyester-containing material can be decomposed without the need for a halogenated solvent, an excess amount of an organic solvent, and an excess amount of a base. Moreover, a dicarboxylic acid diester, which is a monomer, can be obtained as a main depolymerization product with high purity and good yield without requiring additional complicated steps after the decomposition.

A seventh aspect of the present invention is the polyester decomposition method according to the sixth aspect, in which a reaction temperature in the decomposition step is in a range of 30°C to 100°C.

According to the seventh aspect, the polyester contained in the polyester-containing material can be decomposed at a reaction temperature of 100°C or lower.

An eighth aspect of the present invention is the polyester decomposition method according to the sixth aspect, in which the mechanochemical reaction device is a ball mill.

According to the eighth aspect, a polyester contained in a polyester-containing material can be decomposed with smaller amounts of a halogenated solvent, an organic solvent, and a base.

A ninth aspect of the present invention is the polyester decomposition method according to the first or second aspect, in which the polyester-containing material contains at least one of polyester fibers and a film made of a polyester.

According to the ninth aspect, the polyester contained in even a polyester-containing material containing at least one of polyester fibers and a film made of a polyester can be decomposed.

A tenth aspect of the present invention is the polyester decomposition method according to the first or second aspect, in which the polyester-containing material contains a substance other than a polyester.

According to the tenth aspect, the polyester contained in even a polyester-containing material containing a substance other than a polyester can be decomposed.

An eleventh aspect of the present invention is a polyester production method for producing a polyester using the polyester decomposition product obtained by the polyester decomposition method according to the first or second aspect.

According to the eleventh aspect, a new polyester can be produced using as a raw material the polyester decomposition products decomposed by the polyester decomposition method according to the present invention.

A twelfth aspect of the present invention is a polyester decomposition product recovery method for recovering a predetermined polyester decomposition product from the polyester decomposition products obtained by the polyester decomposition method according to the first or second aspect, including a distillation step of distilling the polyester decomposition product, in which the predetermined polyester decomposition product is dimethyl terephthalate, dimethyl 2,6-naphthalenedicarboxylate, or dimethyl 2,5-furandicarboxylate.

According to the twelfth aspect, dimethyl terephthalate, dimethyl 2,6-naphthalenedicarboxylate, or dimethyl 2,5-furandicarboxylate, with high purity (99.0 to 99.9%) can be obtained by simply distilling the resulting polyester decomposition product (solution) containing dimethyl terephthalate, dimethyl 2,6-naphthalenedicarboxylate, or dimethyl 2,5-furandicarboxylate.

### Brief Description of Drawings

[Fig. 1] Fig. 1 shows a ¹H NMR analysis result of dimethyl terephthalate obtained in Example 1.
[Fig. 2] Fig. 2 shows a gas chromatography analysis result of dimethyl terephthalate obtained in Example 1.
[Fig. 3] Fig. 3 shows a ¹H NMR analysis result of dimethyl terephthalate obtained in Example 3.
[Fig. 4] Fig. 4 shows a gas chromatography analysis result of dimethyl terephthalate obtained in Example 3.
[Fig. 5] Fig. 5 is a table showing results in Examples 1 to 6 and Comparative Examples 1 to 4.
[Fig. 6] Fig. 6 is a table showing results in Examples 7 to 9.
[Fig. 7] Fig. 7 is a table showing results in Example 10.
[Fig. 8] Fig. 8 is a table showing results in Examples 11 to 17.
[Fig. 9] Fig. 9 is a table showing results in Examples 18 to 20.
[Fig. 10] Fig. 10 is a table showing results in Examples 21 to 23.
[Fig. 11] Fig. 11 is a table showing results in Examples 19, 24, and 25.
[Fig. 12] Fig. 12 is a table showing results in Examples 26 to 32.
[Fig. 13] Fig. 13 shows a ¹H NMR analysis result of dimethyl terephthalate obtained in Example 50.
[Fig. 14] Fig. 14 shows a gas chromatography analysis result of dimethyl terephthalate obtained in Example 50.
[Fig. 15] Fig. 15 shows a ¹H NMR analysis result of dimethyl terephthalate obtained in Example 51.
[Fig. 16] Fig. 16 shows a gas chromatography analysis result of dimethyl terephthalate obtained in Example 51.
[Fig. 17] Fig. 17 shows a ¹H NMR analysis result of dimethyl terephthalate obtained in Example 52.
[Fig. 18] Fig. 18 shows a gas chromatography analysis result of dimethyl terephthalate obtained in Example 52.
[Fig. 19] Fig. 19 shows a ¹H NMR analysis result of dimethyl terephthalate obtained in Example 53.
[Fig. 20] Fig. 20 shows a gas chromatography analysis result of dimethyl terephthalate obtained in Example 53.
[Fig. 21] Fig. 21 is a table showing decomposition conditions in Examples 36 to 49.
[Fig. 22] Fig. 22 is a table showing decomposition conditions in Comparative Examples 5 to 7, Reference Example 1, and Examples 50 to 53.
[Fig. 23] Fig. 23 is a table showing test results in Examples 36 to 53, Comparative Examples 5 to 7, and Reference Example 1.

### Description of Embodiments

Hereinafter, embodiments of a polyester decomposition method and a polyester production method according to the present invention will be described, but the present invention is not limited to the following embodiments.

### (Embodiment 1)

### <<Decomposition method of polyester>>

The decomposition method of a polyester according to an embodiment of the present invention includes a decomposition step of decomposing a polyester in a material containing a polyester (polyester-containing material) by using (mixing) a base, a monohydric alcohol, and a carbonic acid diester. Note that the polyester-containing material may be one type or two or more types selected from the group consisting of a material containing fibers of a polyester (polyester fibers), a film containing a polyester and a component other than polyesters, and a polyester and a component other than polyesters.

According to the decomposition method of the present embodiment, the polyester can be decomposed at a low temperature using a polyester-containing material of which the polyester has conventionally been difficult to decompose at relatively low temperatures unlike materials such as PET film. Furthermore, a high purity dicarboxylic acid diester, which is a monomer, can be obtained as a main decomposition product (depolymerization product) in a simple step without requiring additional complicated steps after decomposition.

Note that when colored fibers are decomposed by known decomposition methods (methods described in Patent Literatures 6 and 7), only a dicarboxylic acid diester with a purity of about 99% is obtained even if crystallization and distillation are carried out.

On the other hand, according to the present invention, for example, a dicarboxylic acid diester with a purity of 99% or more can be obtained only by a simple washing operation. When distillation is additionally carried out, a dicarboxylic acid diester with a purity of 99.9% or more can be obtained.

### <Polyester-containing material>

In the present embodiment, the polyester-containing material is not particularly limited as long as it contains polyester(s). For example, the polyester-containing material means not only materials within a limited range of having an extremely high polyester content and a form suitable for carrying out a reaction, such as a polyester itself and films made of a polyethylene terephthalate (PET), but also materials within a broader range of having a low polyester content or a form not suitable for carrying out a reaction.

More specifically, examples of the polyester-containing material include a material containing a fiber made of a polyester (polyester fibers), a film containing a polyester and a component other than polyesters, and a lump containing a polyester and a component other than polyesters.

Examples of the material containing a polyester fiber include a polyester fiber (fiber not containing components other than polyesters), a woven fabric of a polyester fiber, a mixture containing a polyester fiber and a component other than polyester fibers, and a woven fabric of a mixture thereof.

Examples of the mixture containing polyester fibers and a component other than polyester fibers include a mixed fiber of a polyester fiber and a fiber other than polyester fibers and a woven fabric of the mixed fiber, a composite mixture containing a polyester fiber and a non-resin component (for example, a colored fiber containing a polyester fiber and a colorant) and a woven fabric of the composite mixture, and a composite mixed fiber containing a polyester fiber, a fiber other than polyester fibers, and a non-resin component (for example, a colored mixed fiber containing a mixed fiber and a colorant) and a woven fabric of the composite mixture. In the present specification, the "non-resin component" means a component that does not correspond to any of polyesters and resins other than polyesters.

Examples of the film containing a polyester and a component other than polyesters include a single-layer film containing a polyester and a component other than polyesters, and a laminated film which is a laminate of a film (polyester film) made of a polyester and a film containing a component other than polyesters.

Examples of the single-layer film containing a polyester and a component other than polyesters include a single-layer film containing both a polyester and a resin other than polyesters and not containing a non-resin component, a single-layer film containing both a polyester and a non-resin component and not containing a resin other than polyesters, and a single-layer film containing all of a polyester, a resin other than polyesters and a non-resin component.

Examples of the laminated film which is a laminate of a film made of a polyester and a film containing a component other than polyesters include a laminated film which is a laminate of a film made of a polyester and a film containing a resin other than polyesters and not containing a polyester and a non-resin component, a laminated film which is a laminate of a film made of a polyester and a film containing a resin other than polyesters and a polyester and not containing a non-resin component, a laminated film which is a laminate of a film made of a polyester and a film containing a resin other than polyesters and a non-resin component and not containing a polyester, a laminated film which is a laminate of a film made of a polyester and a film containing a polyester and a non-resin component and not containing a resin other than polyesters, a laminated film which is a laminate of a film made of a polyester and a film containing all of a polyester, a resin other than polyesters, and a non-resin component, a multilayer film which is a laminate of any one or more of the above-mentioned laminated films and any one or more of the above-mentioned single-layer films, a multilayer film which is a laminate of any two or more of the above-mentioned laminated films, and a multilayer film which is a laminate of any two or more of the above-mentioned single-layer films.

Examples of the lumps containing a polyester and a component other than polyesters include a lump containing both a polyester and a resin other than polyesters and not containing a non-resin component, a lump containing both a polyester and a non-resin component and not containing a resin other than polyesters, and a lump containing all of a polyester, a resin other than polyesters, and a non-resin component.

The resin other than polyesters can be arbitrarily selected depending on the purpose, and is not particularly limited. For example, in terms of high versatility and high applicability of the present invention, examples of the resin other than polyesters include a polyolefin such as a polyethylene and a polypropylene, a cellulose, a polyamide such as a nylon, a polyurethane, and an acrylic resin. These resins may be, for example, in the form of film (resin film) or fiber (resin fiber) .

Examples of the fibers other than polyester fibers include fibers of resins other than the above-mentioned polyester, cotton, and rayon.

The non-resin component can be arbitrarily selected according to the purpose, and is not particularly limited. For example, in terms of high versatility and high applicability of the present invention, examples of the non-resin component include an inorganic component (inorganic compound) such as aluminum, and a colorant such as a dye and a pigment.

The fiber diameters of the polyester fiber and the fiber other than the polyester fiber in the material containing the polyester fiber are not particularly limited, and may be, for example, 0.1 µm to 200 µm, or may be 1 µm to 50 µm.

The thickness of the film containing a polyester and a component other than polyesters is not particularly limited, and may be, for example, 0.5 µm to 1,000 µm, or may be 1 µm to 500 µm. Here, when the film is the laminated film described above, the thickness of the film means the thickness of the entire laminated film.

More specific examples of the material containing polyester fibers include fabrics of various clothes and fibers as a material of fabrics. The fabrics may be, for example, small pieces such as cut pieces generated during the production of various clothes or cut pieces of various used clothes. The fibers as a material of fabrics may be, for example, small pieces such as cut pieces generated during the production of fabrics or cut pieces generated from used fabrics.

More specific examples of the film containing a polyester and a component other than polyesters include a packaging film, small pieces such as cut pieces generated during the production of a packaging film, and small pieces such as cut pieces of a used packaging film.

More specific examples of the lump containing a polyester and a component other than polyesters include pellets and flakes containing polyester and a component other than polyester. The maximum diameter of the lump is not particularly limited, and may be, for example, 0.1 mm to 10 mm. Here, the "maximum diameter of the lump" refers to the maximum value of the length of a line segment connecting two different points on the surface of the lump.

As the polyester-containing material used for the polyester decomposition, one type thereof may be used solely or two or more types thereof may be used. When two or more types of the polyester-containing materials are used, the combination and ratio thereof can be arbitrarily selected depending on the purpose. That is, the polyester-containing material used for the polyester decomposition is one type or two or more types selected from the group consisting of a material containing polyester fibers, a film containing a polyester and a component other than polyesters, and a lump containing a polyester and a component other than polyesters.

When the polyester-containing material is a material containing polyester fibers, the material containing polyester fibers is preferably one type or two or more types selected from the group consisting of a polyester fiber, a woven fabric of polyester fibers, a mixture containing polyester fibers and a component other than polyester fibers, and a woven fabric of the mixture.

When the polyester-containing material is a film containing a polyester and a component other than polyesters, the film containing a polyester and a component other than polyesters is preferably one or both of a single-layer film containing a polyester and a component other than polyesters, and a laminated film which is a laminate of a film made of a polyester and a film containing a component other than polyesters.

In the polyester-containing material, the ratio of the content (parts by mass) of the polyester to the total mass (parts by mass) of the polyester-containing material ([content (parts by mass) of the polyester contained in the polyester-containing material] / [total mass (parts by mass) of the polyester-containing material] × 100) (polyester content ratio) can be arbitrarily selected according to the purpose, and is not particularly limited. In particular, the polyester content ratio may be 20% by mass or more and 100% by mass or less, or 40% by mass or more and 100% by mass or less, preferably 40% by mass or more and less than 100% by mass, or 55% by mass or more and 100% by mass or less, more preferably 55% by mass or more and less than 100% by mass, or 70% by mass or more and 100% by mass or less, particularly preferably 70% by mass or more and less than 100% by mass.
The higher the polyester content ratio is, the more the amount of monomers (a dicarboxylic acid diester described below) produced from the polyester-containing material per unit mass by the polyester decomposition increases.

### [Polyester]

The polyester (the polyester to be decomposed) in the polyester-containing material is not particularly limited as long as it is an oligomer or a polymer that generates, by decomposition, a glycol and a component having two functional groups capable of condensation reaction with the glycol in one molecule.

The polyester may be an aromatic polyester having only an aromatic group (a divalent group having a structure obtained by removing one hydrogen atom from each of two carbon atoms forming an aromatic ring skeleton in an aromatic compound) in its main chain having an ester bond, an aliphatic polyester having no aromatic group (having an aliphatic group and having no aromatic group) in its main chain, or a polyester having both an aromatic group and an aliphatic group in its main chain. The aromatic polyester may have only a divalent aromatic hydrocarbon group (arylene group) as an aromatic group, only a divalent aromatic heterocyclic group (heteroarylene group), or both a divalent aromatic hydrocarbon group and a divalent aromatic heterocyclic group. Examples of the hetero atom contained in the aromatic heterocyclic group include an oxygen atom and a nitrogen atom.

In general, the reactivity of a substrate in the transesterification reaction depends on the structure of the carboxylic acid side and the structure of the alcohol side. As will be described later, since even polyesters with aromatic structures, such as a benzene ring or a naphthalene ring (a polyethylene terephthalate, a polybutylene terephthalate, and the like) as the structure of the carboxylic acid side can be satisfactorily decomposed, it can be reasonably inferred that polyesters even with other aromatic structures, such as furan, can also be decomposed in the same manner. Similarly, since polyesters with a structure of ethylene glycol (a polyethylene terephthalate or the like) or 1,4-butanediol (a polybutylene terephthalate or the like) as the structure of the alcohol side can be satisfactorily decomposed, it can be reasonably inferred that polyesters even with other divalent alcohol structures, such as 1,3-propanediol, can be decomposed in the same manner.

The polyester is preferably an aromatic polyester in terms of higher effects of the present invention and high versatility, and is more preferably a polyethylene terephthalate (PET), a polybutylene terephthalate (PBT), a polytrimethylene terephthalate (PTT), a polyethylene naphthalate (PEN), a polybutylene naphthalate (PBN), or a polyethylene furanoate (PEF, also known as a polyethylene furandicarboxylate).

By the polyester decomposition, a dicarboxylic acid diester specified according to the type of the polyester and the monohydric alcohol is produced from the polyester as a monomer.

For example, a terephthalic acid diester is produced from a polyethylene terephthalate, a polybutylene terephthalate, and a polytrimethylene terephthalate. A naphthalene dicarboxylic acid diester (2,6-naphthalene dicarboxylic acid diester) is produced from a polyethylene naphthalate and a polybutylene naphthalate. A furandicarboxylic acid diester (2,5-furandicarboxylic acid diester) is produced from a polyethylene furanoate.

For example, when methanol is used as the monohydric alcohol, a dicarboxylic acid dimethyl ester is produced as the dicarboxylic acid diester.

As the polyester used for the decomposition, one type thereof may be used solely or two or more types thereof may be used. When two or more types of the polyesters are used, the combination and ratio thereof can be arbitrarily selected depending on the purpose.

### <Base>

The base is not particularly limited, and may be either an inorganic base or an organic base, but is preferably an alkali metal carbonate or an alkali metal hydroxide that reacts with a monohydric alcohol to produce an alkali metal alkoxide, or an alkali metal alkoxide.

In general, in the transesterification reaction, an alkoxide anion, which serves as a nucleophile, attacks the carbonyl group of an ester to produce an intermediate of a quaternary carbon type, and then the other alkoxide anion is released, whereby the reaction proceeds. Alkoxide anions are generated by the reaction of the corresponding alcohol with a base, and so any base capable of deprotonating the alcohol can be used.

Examples of the inorganic base include an alkali metal hydroxide such as lithium hydroxide, sodium hydroxide, and potassium hydroxide, an alkali metal oxide such as lithium oxide, sodium oxide, and potassium oxide, an alkaline earth metal hydroxide such as calcium hydroxide and magnesium hydroxide, and an alkaline earth metal oxide such as calcium oxide and magnesium oxide.

Examples of the organic base include an alkali metal alkoxide such as lithium methoxide, lithium ethoxide, lithium tert-butoxide, sodium methoxide, sodium ethoxide, sodium tert-butoxide, potassium methoxide, potassium ethoxide, and potassium tert-butoxide, an alkaline earth metal alkoxide such as calcium dimethoxide, calcium diethoxide, calcium di-tert-butoxide, magnesium dimethoxide, magnesium diethoxide, and magnesium di-tert-butoxide, and a nitrogen-containing organic base (an organic base having a nitrogen atom) such as trimethylamine, triethylamine, tributylamine, trioctylamine, 1,5,7-triazabicyclo[4.4.0]deca-5-ene (abbreviation: TBU), 1,8-diazabicyclo[5.4.0]undeca-7-ene (abbreviation: DBU), 1,3-dimethylimidazol-2-ylidene, and 1,3-dicyclohexylimidazol-2-ylidene.

The alkali metal alkoxide is preferably lithium methoxide, sodium methoxide or potassium methoxide, and more preferably sodium methoxide, from the viewpoint that the polyester decomposition proceeds at a higher rate.

The nitrogen-containing organic base is preferably 1,5,7-triazabicyclo[4.4.0]deca-5-ene from the viewpoint that the polyester decomposition proceeds at a higher rate.

As the base used for the polyester decomposition, one type thereof may be used solely or two or more types thereof may be used. When two or more types of the bases are used, the combination and ratio thereof can be arbitrarily selected depending on the purpose.

The base is particularly preferably an alkali metal alkoxide from the viewpoint that the polyester decomposition proceeds at a particularly high rate.

The use amount of the base at the time of the polyester decomposition is preferably 0.1 to 10 parts by mass, more preferably 0.5 to 6 parts by mass, and may be, for example, any of 0.5 to 3 parts by mass and 3 to 6 parts by mass, relative to 100 parts by mass of the polyester in the polyester-containing material. When the use amount of the base is equal to or more than the lower limit value, the polyester decomposition proceeds at a higher rate. When the use amount of the base is equal to or less than the upper limit value, excessive use of the base is suppressed. That is, the use amount of the base at the time of the polyester decomposition is preferably a catalytic amount (the base serves as a catalyst).

### <Monohydric alcohol>

The monohydric alcohol undergoes a transesterification reaction with the polyester in the polyester-containing material during decomposition. That is, the reaction between the polyester and the monohydric alcohol during polyester decomposition generates a glycol corresponding to one monomer among the raw materials used in the production of the polyester, and also generates a dicarboxylic acid diester corresponding to the other monomer or a derivative thereof.

The monohydric alcohol is not particularly limited. The reaction efficiency of the decomposition reaction according to the present invention is controlled by capturing released dihydric alcohols by an excess carbonic acid diester. Therefore, as will be described later, from the fact that the reaction using methanol and a carbonic acid diester proceeds efficiently, it can be reasonably inferred that the reaction proceeds efficiently in a combination of other monohydric alcohols and dialkyl carbonate as well.

For example, the monohydric alcohol may be an aliphatic alcohol or an aromatic alcohol, but is preferably an aliphatic alcohol (a saturated aliphatic alcohol or an unsaturated aliphatic alcohol), and more preferably a saturated aliphatic alcohol, from the viewpoint that the polyester decomposition proceeds at a higher rate.

Examples of the saturated aliphatic alcohol include alcohols having 1 to 4 carbon atoms such as methanol, ethanol, 1-propanol (n-propyl alcohol), 2-propanol (isopropyl alcohol), 1-butanol (n-butyl alcohol), 2-methyl-1-propanol (isobutyl alcohol), 2-butanol (sec-butyl alcohol), and 2-methyl-2-propanol (tert-butyl alcohol).

As the monohydric alcohol used for the polyester decomposition, one type thereof may be used solely or two or more types thereof may be used. When two or more types of the monohydric alcohols are used, the combination and ratio thereof can be arbitrarily selected depending on the purpose. The monohydric alcohol is particularly preferably methanol from the viewpoint that the polyester decomposition proceeds at a particularly high rate.

The use amount of the monohydric alcohol at the time of the polyester decomposition is not particularly limited, and for example, when the polyester is a polyethylene terephthalate (PET), the use amount of the monohydric alcohol is preferably 30 to 200 parts by mass, and more preferably 50 to 160 parts by mass, relative to 100 parts by mass of PET in the polyester-containing material. When the polyester is a polybutylene terephthalate (PBT), the use amount of the monohydric alcohol is preferably 10 to 180 parts by mass, and more preferably 20 to 140 parts by mass, relative to 100 parts by mass of PBT in the polyester-containing material. When the use amount of the monohydric alcohol is equal to or more than these lower limit values, the effect obtained by using the monohydric alcohol becomes higher. On the other hand, when the use amount of monohydric alcohol is less than or equal to these upper limit values, excessive use of the monohydric alcohol is suppressed.

### <Carbonic acid diester>

The carbonic acid diester has an effect of producing a cyclic compound or a chain compound by reacting with a glycol generated from the polyester during polyester decomposition, and deviating the reaction at the time of the polyester decomposition, that is, the equilibrium between the depolymerization reaction and the polymerization reaction of the polyester so that the depolymerization reaction becomes dominant, thereby improving the production rate of the target monomer. The carbonic acid diester functions as a glycol scavenger.

As a reaction product of a carbonic acid diester and a glycol, the following compounds can be produced: a cyclic compound (for example, a cyclic compound (61) described later) which is a reaction product of one molecule of a carbonic acid diester and one molecule of a glycol; a chain compound (for example, a first chain compound (62) described later) which is a reaction product of one molecule of a carbonic acid diester and one molecule of a glycol; and a chain compound (for example, a second chain compound (63) described later) which is a reaction product of one molecule of a carbonic acid diester and two molecules of a glycol. Which of these cyclic compound and linear compounds is formed is determined mainly by the influence of the type (e.g., size) of the glycol. For example, a reaction product of a carbonic acid diester and ethylene glycol is mainly a cyclic compound (more specifically, ethylene carbonate), which is a reaction product of one molecule of carbonic acid diester and one molecule of ethylene glycol.

Examples of the carbonic acid diester include a dialkyl carbonate and a diaryl carbonate. The two alkyl groups bonded to the oxygen atom in the dialkyl carbonate may be the same as or different from each other. The two aryl groups bonded to the oxygen atom in the diaryl carbonate may be the same as or different from each other.

The alkyl group in the dialkyl carbonate may be linear, branched or cyclic. When the alkyl group is cyclic, it may be monocyclic or polycyclic.

The number of carbon atoms of the linear or branched alkyl group in the dialkyl carbonate is preferably 1 to 8. Examples of such an alkyl group include a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, a 1-methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, and an isooctyl group.

In particular, the number of carbon atoms of the alkyl group is more preferably 1 to 4, and still more preferably 1 or 2.
Examples of such more preferred dialkyl carbonates include dimethyl carbonate, diethyl carbonate, and methyl ethyl carbonate.

The aryl group in the diaryl carbonate may be either monocyclic or polycyclic. The number of carbon atoms of the aryl group in the diaryl carbonate is preferably 6 to 10, and examples of such an aryl group include a phenyl group, a 1-naphthyl group, a 2-naphthyl group, an o-tolyl group, a p-tolyl group, a 2,3-xylyl group (2,3-dimethylphenyl group), a 2,4-xylyl group (2,4-dimethylphenyl group), a 2,5-xylyl group (2,5-dimethylphenyl group), a 2,6-xylyl group (2,6-dimethylphenyl group), a 3,4-xylyl group (3,4-dimethylphenyl group), and 3,5-xylyl group (3,5-dimethylphenyl group). Examples of more preferred diaryl carbonates include a diphenyl carbonate.

As the carbonic acid diester used for the polyester decomposition, one type thereof may be used solely or two or more types thereof may be used. When two or more types of the carbonic acid diesters are used, the combination and ratio thereof can be arbitrarily selected depending on the purpose.

From the viewpoint that the polyester decomposition proceeds at a higher rate, the carbonic acid diester is more preferably one type or two or more types selected from the group consisting of dimethyl carbonate, diethyl carbonate, methyl ethyl carbonate, and diphenyl carbonate, and particularly preferably dimethyl carbonate.

The use amount of the carbonic acid diester at the time of the polyester decomposition is preferably 100 to 5,000 parts by mass, more preferably 100 to 1,000 parts by mass, and may be, for example, 100 to 500 parts by mass, relative to 100 parts by mass of the polyester in the polyester-containing material. When the use amount of the carbonic acid diester is equal to or more than the lower limit value, the effect obtained by using the carbonic acid diester becomes higher. When the use amount of the carbonic acid diester is equal to or less than the upper limit value, excessive use of the carbonic acid diester is suppressed.

### <Solvent>

When the polyester is decomposed, a solvent which does not correspond to any of bases, monohydric alcohols, and carbonic acid diesters may be used. In the present embodiment, although the polyester decomposition proceeds efficiently without using a solvent, the use of a solvent if necessary may improve the handleability of a blend of the respective raw materials such as a reaction solution, or the polyester decomposition may proceed more efficiently.

In the present specification, unless otherwise specified, the term "solvent" is a concept including both a component in a liquid state at room temperature for dissolving a solute and a component in a liquid state at room temperature that functions as a dispersion medium for dispersing a dispersoid. The term "normal temperature" means a temperature at which a material is not particularly cooled or heated, that is, a normal temperature, and examples thereof include a temperature of 15°C to 25°C.

Here, the solvent is preferably an organic solvent. Examples of the organic solvents include an aromatic hydrocarbon such as toluene, an ether such as tetrahydrofuran, an alkane such as n-hexane, a halogenated hydrocarbon such as chloroform and dichloromethane, an amide such as dimethylformamide, and a sulfoxide such as dimethylsulfoxide.

As the solvent used for the polyester decomposition n, one type thereof may be used solely or two or more types thereof may be used. When two or more types of the solvents are used, the combination and ratio thereof can be arbitrarily selected depending on the purpose.

When a solvent is used, the use amount of the solvent at the time of the polyester decomposition is preferably 1 to 100,000 parts by mass, and more preferably 1 to 10,000 parts by mass, relative to 100 parts by mass of the polyester-containing material. When the use amount of the solvent is equal to or more than the lower limit value, the effect obtained by using the solvent becomes higher. When the use amount of the solvent is equal to or less than the upper limit value, excessive use of the solvent is suppressed.

### <Other components>

At the time of the polyester decomposition, as long as the effect of the present invention is not impaired, other components that do not fall under any of the polyester-containing material, the base, the monohydric alcohol, the carbonate diester, and the solvent may be used.

The other components can be arbitrarily selected depending on the purpose, and are not particularly limited. As the other components used for the polyester decomposition, one type thereof may be used solely or two or more types thereof may be used. When two or more types of the other components are used, the combination and ratio thereof can be arbitrarily selected depending on the purpose.

At the time of the polyester decomposition, the ratio (parts by mass) of the total use amount of the polyester-containing material, the base, the monohydric alcohol, and the carbonic acid diester to the total use amount (parts by mass) of the components other than the solvent ([the use amount (parts by mass) of the polyester-containing material)] + [the use amount (parts by mass) of the base] + [the use amount (parts by mass) of the monohydric alcohol] + [the use amount (parts by mass) of the carbonic acid diester]) / [the total use amount (parts by mass) of the components other than the solvent] × 100) is preferably 80% by mass to 100% by mass, more preferably 90% by mass to 100% by mass, and still more preferably 95% by mass to 100% by mass, and may be any of, for example, 97% by mass to 100% by mass and 99% by mass to 100% by mass. When the ratio is equal to or more than the lower limit value, the polyester can be decomposed more efficiently.

Here, the total use amount (parts by mass) of the components other than the solvent at the time of the polyester decomposition has the same meaning as the total use amount (parts by mass) of the polyester-containing material, the base, the monohydric alcohol, the carbonic acid diester, and the other components at the time of the decomposition.

### <Reaction conditions of decomposition process>

The polyester in the polyester-containing material can be decomposed by blending (mixing) a base, a monohydric alcohol, a carbonic acid diester, a polyester-containing material, a solvent, if necessary, and other components, if necessary.

The blending order of these respective raw materials is not particularly limited, but it is preferable to first prepare a raw material composition which is a blend of a base, a monohydric alcohol, a carbonic acid diester, a solvent, if necessary, and other components, if necessary (for example, a blend of all raw materials other than the polyester-containing material), and then mixing the raw material composition with the polyester-containing material. With such a blending order, the polyester decomposition proceeds at a higher rate.

In the case where the blend amount of the base in the raw material composition is a catalytic amount (the base serves as a catalyst), the raw material composition may be referred to as a "catalyst composition" in the present specification.

The reaction temperature during polyester decomposition (reaction temperature in the decomposition step) can be appropriately adjusted in consideration of the types of the raw materials to be used and the like, and the decomposition may be carried out at room temperature (normal temperature) or under heating conditions. The heating device is not particularly limited, and may include devices such as a heater.

The reaction temperature during polyester decomposition (reaction temperature in the decomposition step) is preferably 20°C or higher, and may be, for example, any of 40°C or higher and 60°C or higher. The reaction temperature during polyester decomposition is preferably 150°C or lower, and may be, for example, any of 100°C or lower and 70°C or lower.

In an embodiment, the reaction temperature during polyester decomposition (the reaction temperature in the decomposition step) is, for example, preferably 20 to 150°C and more preferably 50 to 120°C. For example, by adjusting the reaction conditions other than the reaction temperature, it is possible to decompose the polyester at a sufficiently high rate even at a reaction temperature of 20 to 70°C.

According to the present embodiment, as described above, in spite of not only the use of PET films and the like, but also the use of the polyester-containing materials of which the polyester has conventionally been difficult to decompose at relatively low temperatures, polyesters can be decomposed at a low temperature of such as 150°C or lower. As a result, unlike a case where the polyester is decomposed at a high temperature, the amount of by-products produced during decomposition can be reduced, and the coloring of the dicarboxylic acid diester (monomer), which is a main decomposition product, can also be reduced.

The polyester may be decomposed under any of atmospheric pressure, reduced pressure, and pressurization. The polyester may be decomposed either under an atmosphere or an inert gas atmosphere.

Furthermore, the reaction time at the time of the polyester decomposition can be appropriately adjusted in consideration of other reaction conditions such as the reaction temperature, and is not particularly limited. The reaction time at the time of the polyester decomposition is not particularly limited as long as it is 0.5 to 24 hours, but is preferably 0.5 to 12 hours, and more preferably 1 to 8 hours.

In the present embodiment, for example, it can be determined that the disappearance time of the polyester is the end time of the decomposition. Therefore, the time required for the polyester to disappear can be regarded as the reaction time of the decomposition. The time required for the polyester to disappear can also be determined, for example, by the time required for the mass reduction of the polyester-containing material to cease.

In the blend immediately after blending the respective raw materials, the unreacted polyester-containing material does not dissolve and remains undissolved in the other liquid components. In a blend (reactants) in which decomposition of the polyester is in progress, both the unreacted polyester-containing material and the polyester-containing material during the reaction of the polyester or after the reaction typically do not dissolve, and so they remain undissolved in the other liquid components. On the other hand, glycols and dicarboxylic acid diesters, which are reaction products of the decomposition of the polyester, are typically dissolved in the liquid components.

Such a blend can be stirred by a known method during polyester decomposition, and, for example, a method of stirring it by rotating a magnetic stirrer or a stirring blade, a method of stirring it using a ball mill, or the like can be employed.

When the polyester is decomposed, for example, a reaction vessel having a flat bottom surface can be used to increase the contact area of the polyester-containing material, which is insoluble in the liquid components, with the liquid components. As a result, the use amount of raw materials such as the base, the monohydric alcohol, and the carbonic acid diester can be reduced more than that in the case of using other reaction vessels, and the polyester can be decomposed more efficiently.

### <Post-processing condition and taking-out condition>

After the end of the above-described step of decomposing a polyester, post-processing is carried out by a known method, so that a dicarboxylic acid diester, which is one main decomposition product, can be taken out with high purity.

For example, a high-purity dicarboxylic acid diester can be obtained by decomposing a polyester, followed by filtration of the resulting reaction products, distillation (condensation) of volatile matter, and washing the resulting solid matter with methanol and water. Furthermore, the resulting dicarboxylic acid diester may be purified by crystallization, distillation, or the like, if necessary.

The reaction product between glycol as the other main decomposition product and the carboxylic acid diester can also be taken out by appropriately adjusting the post-processing conditions and the taking-out conditions similarly to those in the case of the dicarboxylic acid diester.

According to the present embodiment, decomposition is possible at a low temperature of, for example, 150°C or lower as previously explained. Therefore, the impurity content and coloring of the dicarboxylic acid diester can be reduced, unlike a case where decomposition is carried out at a high temperature. Thus, a high-purity dicarboxylic acid diester (monomer) in which coloring is suppressed is obtained in a simplified step of washing with methanol and water as described above without requiring additional complicated steps. In addition, even if the polyester-containing material originally contains a colorant, the coloring of the dicarboxylic acid diester (monomer) derived from the colorant can also be reduced in a simplified step of washing with methanol and water as described above.

On the contrary, when decomposition is carried out at a high temperature by a known method, a complicated step including decoloring with a high boiling point solvent and decomposition of a dye with an oxidant is necessary in order to obtain high-purity monomers.

### <Method for producing polyester>

Among the polyester decomposition products obtained by the above-described polyester decomposition method according to an embodiment of the present invention, the dicarboxylic acid diester can be reused for polyester production. For example, Japanese Patent Application Laid-Open No. 2011-26437 discloses that dimethyl terephthalate (DMT), which is a decomposition product of polyethylene terephthalate, is reused for polyethylene terephthalate production.

On the other hand, among the polyester decomposition products, a reaction product of a glycol and a carbonic acid diester can be converted into a glycol and a carbonic acid diester by a reaction with an alcohol. The regenerated carbonic acid diester can be reused for polyester decomposition, and the regenerated glycol can be reused for polyester production. For example, when a polyester having a structure considered to be derived from ethylene glycol is decomposed, ethylene carbonate is produced as a reaction product of ethylene glycol and a carbonic acid diester. Japanese Patent No. 4236208 discloses the conversion of ethylene carbonate into ethylene glycol and a carbonic acid diester by reaction with an alcohol.

Therefore, by using the polyester decomposition method according to an embodiment of the present invention, the polyester can be continuously produced in a closed-loop system without introducing new production raw materials in a three-system chemical reaction of decomposition of a polyester, regeneration of a glycol and a carbonic acid diester from a reaction product of the glycol and the carbonic acid diester, and production of a polyester by reaction of a dicarboxylic acid diester and a glycol.

That is, the method for producing a polyester according to the present embodiment includes a step of producing a polyester using polyester decomposition products (dicarboxylic acid diester, glycol, or the like) obtained by the above-described method for decomposing a polyester according to the present embodiment.

The method for producing a polyester of the present embodiment is highly suitable for reuse of raw materials, and can decompose the polyester at a low temperature, and therefore has extremely high industrial utility value.

The continuous production of a polyester in such a closed-loop system is represented by the following formula (chemical reaction formula). That is, according to the present embodiment, a dicarboxylic acid diester represented by the following general formula (3) (in the present specification, sometimes referred to as "dicarboxylic acid diester (3)") and a glycol represented by the following general formula (4) (in the present specification, sometimes referred to as "glycol (4)") are produced by the reaction of a polyester represented by the following general formula (1) (in the present specification, sometimes referred to as "polyester (1)") and a monohydric alcohol represented by the following general formula (2) (in the present specification, sometimes referred to as "monohydric alcohol (2)") in the copresence of a base. Then, by the reaction of the glycol (4) and a carbonic acid diester represented by the following general formula (5) (in the present specification, sometimes referred to as "carbonic acid diester (5)"), one type or two or more types (in the present specification, these cyclic compounds and chain compounds are sometimes referred to as "compound (6)") selected from the group consisting of a cyclic compound represented by the following general formula (61) (in the present specification, sometimes referred to as "cyclic compound (61)"), a first chain compound represented by the following general formula (62) (in the present specification, sometimes referred to as "first chain compound (62)"), and a second chain compound represented by the following general formula (63) (in the present specification, sometimes referred to as "second chain compound (63)") are produced.

By the reaction of these compounds (6) with an alcohol represented by the following general formula (7) (in the present specification, sometimes referred to as "alcohol (7)"), the glycol (4) and the carbonic acid diester (5) are regenerated, and by the reaction of the regenerated glycol (4) and dicarboxylic acid diester (3), the polyester (1) is again produced. The regenerated carbonic acid diester (5) can be reused at the time of the depolymerization (decomposition) of the polyester (1).

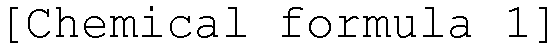

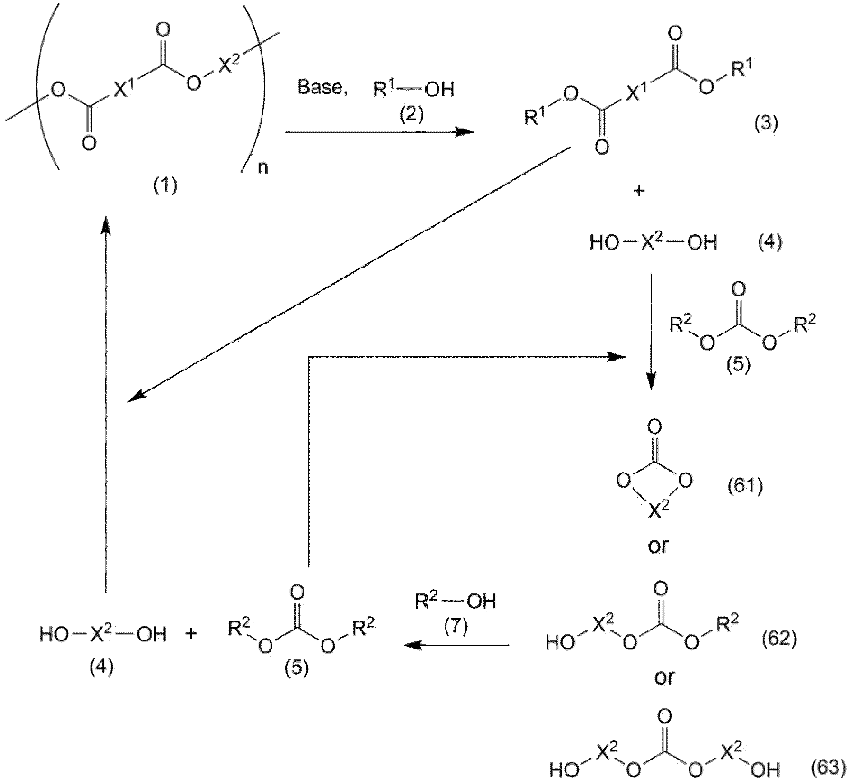

(In the formula, X¹ and X² are each independently a divalent organic group, R¹ and R² are each independently a monovalent organic group, and n is an integer of 2 or more.)

Here, the polyester (1) is a polyester in the polyester-containing material described above. The monohydric alcohol (2) is a monohydric alcohol used in the step of depolymerizing a polyester described above. The dicarboxylic acid diester (3) is a dicarboxylic acid diester which is one of the main depolymerization products described above. The glycol (4) is a glycol which is the other main depolymerization product described above. The carbonic acid diester (5) is a carbonic acid diester used in the step of depolymerizing the polyester described above. The compounds (6) (the cyclic compound (61), the first chain compound (62), and the second chain compound (63)) are reaction products of a carbonic acid diester and a glycol produced in the step of depolymerizing the polyester described above. The alcohol (7) is a raw material used for the regeneration of the carbonic acid diester and the glycol, and determines the structure of the carbonic acid diester.

In the formula, X¹ is a divalent organic group, and may be, for example, any of an aromatic group, an aliphatic group, or a group having both an aromatic group and an aliphatic group.

Examples of the preferred X¹ include an aromatic group such as a benzene-1,4-diyl group (1,4-phenylene group), a naphthalene-2,6-diyl group (2,6-naphthylene group), and a furan-2,5-diyl group (2,5-furanylene group).

In the formula, X² is a divalent organic group, and may be, for example, any of an aromatic group, an aliphatic group, or a group having both an aromatic group and an aliphatic group.

Examples of the preferred X² include an alkylene group such as an ethylene group (-CH₂CH₂-), a 1,3-propylene group (trimethylene group, -CH₂CH₂CH₂-), and a 1,4-butylene group (tetramethylene group, -CH₂CH₂CH₂CH₂-). X² is more preferably an alkylene group having 2 to 4 carbon atoms.

In the formula, R¹ is a monovalent organic group, and may be, for example, either an aromatic group or an aliphatic group, but is preferably an aliphatic group (a saturated aliphatic group or an unsaturated aliphatic group).

Examples of the preferred R¹ include a monovalent saturated aliphatic group (i.e., an alkyl group) such as a methyl group, an ethyl group, an n-propyl group, an isopropyl group, an n-butyl group, an isobutyl group, a sec-butyl group, and a tert-butyl group. R¹ is more preferably a monovalent saturated aliphatic group having 1 to 4 carbon atoms (i.e., an alkyl group).

In the formula, R² is a monovalent organic group, and examples of R² include the same as those of R¹.

In the formula, n is an integer of 2 or more, preferably an integer of 20 to 1,000.

Hereinafter, the present invention will be described in detail with reference to specific examples. However, the present invention is not limited to the following examples. The units "ppm" shown below are all based on the mass ratio.

### <<Decomposition of polyester>>

### <Decomposition of polyester fibers>

### [Example 1]

Into a 300-mL eggplant flask, sodium methoxide (NaOCH₃) (manufactured by FUJIFILM Wako Pure Chemical Corporation, the same applies below, 0.58 g, 10.4 mmol, 2.9% by mass relative to the mass of polyester fibers described later) was added. Furthermore, methanol (manufactured by Kishida Chemical Co., Ltd., the same applies below, 13 mL, 51.4% by mass relative to the mass of polyester fibers described later) and dimethyl carbonate (DMC) (manufactured by Tokyo Chemical Industry Co., Ltd., the same applies below, 100 mL, 535% by mass relative to the mass of polyester fibers described later) were added. These blended components were uniformly dissolved to prepare a catalyst composition. A white coat for testing (the ratio of polyester fibers in all fibers: 100% by mass) was cut into a size of about 2 cm × 2 cm. A plurality of these cut pieces (20 g) was added to the above obtained catalyst composition in the eggplant flask.

Here, the ratio (% by mass) of polyester fibers in all fibers has the same meaning as the above-described ratio of the content (parts by mass) of polyester to the total mass (parts by mass) of the polyester-containing material in the polyester-containing material. This applies to subsequent other Examples and Comparative Examples.

Next, the mixture of the catalyst composition and the cut pieces was stirred at 50°C for 3 hours while rotating a stirring bar in the eggplant flask using a magnetic stirrer and an oil bath to decompose the polyester fibers. Next, dimethyl carbonate (30 mL) was added to this reaction product in the eggplant flask, so that the deposited object was dissolved. The contents in the eggplant flask were filtered during hot state while maintaining the temperature at 50°C without cooling. The filtrate was concentrated to recover dimethyl carbonate and methanol, and the residue was washed with methanol (50 mL) and water (50 mL) and dried to obtain dimethyl terephthalate (18.5 g).

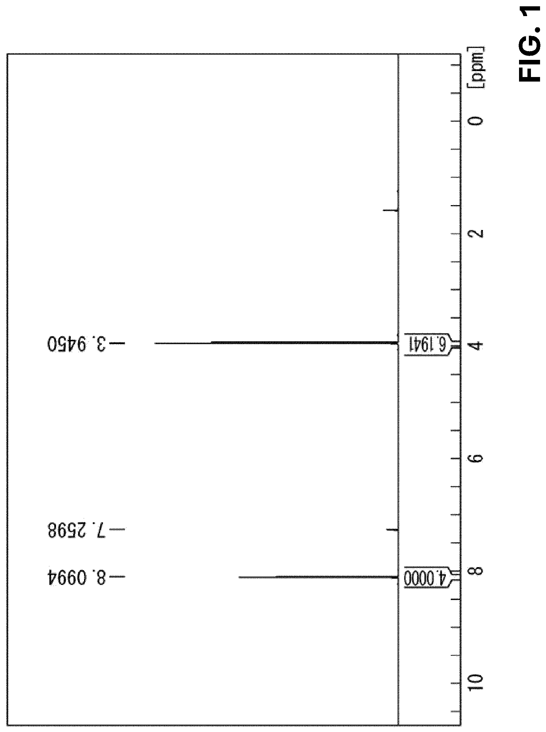

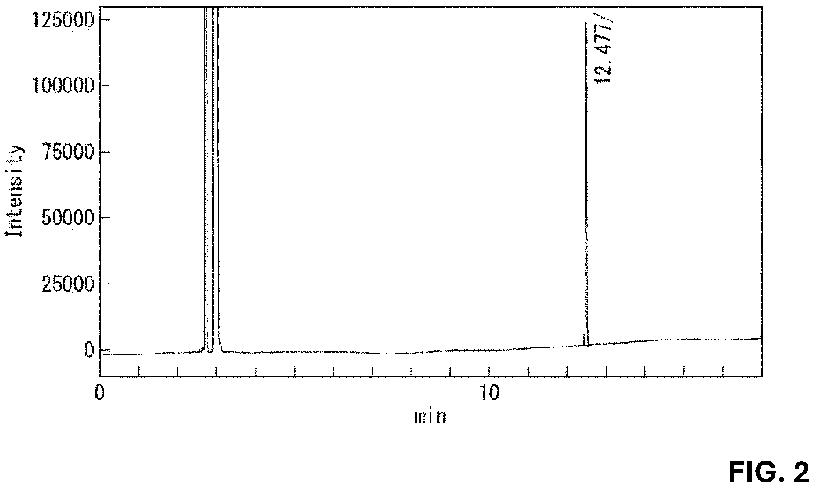

The resulting dimethyl terephthalate was analyzed by ¹H NMR (device: "Bruker Avance III (registered trademark)" 600 MHz manufactured by BRUKER Co., the same applies below) and gas chromatography (device: "GC-2000" manufactured by Shimadzu Corporation, the same applies below). As a result, it was confirmed that dimethyl terephthalate with a purity of 99% by mass or more was obtained. The analysis result by ¹H NMR is shown in Fig. 1, and the analysis result by gas chromatography is shown in Fig. 2.

### <Decomposition of polyester fibers in mixed fibers containing polyester fibers and cotton>

### [Example 2]

A white coat for chemical experiments (the ratio of polyester fibers in all fibers: 65% by mass, the ratio of cotton: 35% by mass) was cut into a size of about 2 cm × 2 cm.

Here, the ratio (% by mass) of cotton in all fibers refers to the ratio of the content (parts by mass) of cotton to the total mass (parts by mass) of the polyester-containing material in the polyester-containing material. This applies to fibers other than cotton in subsequent other Examples.

Next, dimethyl terephthalate (11.7 g) was obtained by decomposing polyester fibers in cut pieces in the same manner as that in Example 1, except that a plurality of previously dried cut pieces (20 g) of this white coat for chemical experiments was used instead of the above-described plurality of cut pieces (20 g) of the white coat for testing, and that the use amount of sodium methoxide was changed to 0.38 g instead of 0.58 g. In the present Example, the use amount of methanol at the time of the decomposition was 79.1% by mass relative to the amount of polyester fibers in the mixed fibers.

The resulting dimethyl terephthalate was analyzed by ¹H NMR and gas chromatography in the same manner as that in Example 1, and similar data to those in Example 1 was obtained. Thus, it was confirmed that dimethyl terephthalate with a purity of 99% by mass or more was obtained.

### <Decomposition of polyester fibers in colored fibers containing polyester fibers and colorant>

### [Example 3]

Colored clothing (the ratio of polyester fibers in all fibers: 100% by mass, including a dye of red, black, blue, or the like) was cut into a size of about 2 cm × 2 cm. Dimethyl terephthalate (18.4 g) was obtained by decomposing polyester fibers in the cut pieces in the same manner as that in Example 1, except that the plurality of cut pieces (20 g) of the colored clothing was used instead of the above-described plurality of cut pieces (20 g) of the white coat for testing. In the present Example, the use amount of methanol at the time of the decomposition was 51.4% by mass relative to the amount of polyester fibers in the colored fibers.

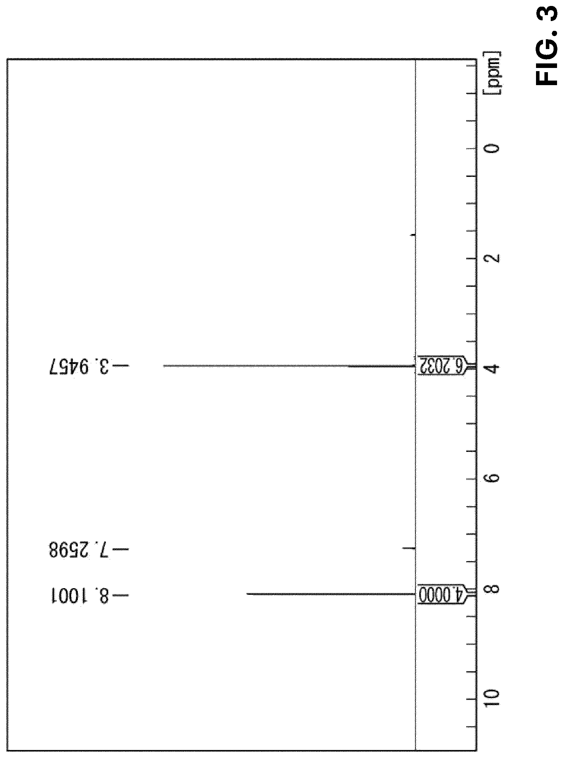

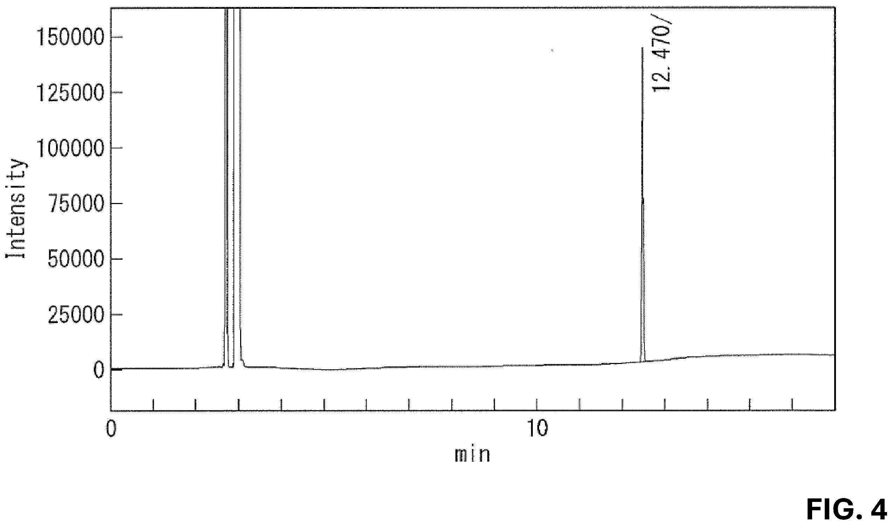

The resulting dimethyl terephthalate was analyzed by ¹H NMR and gas chromatography in the same manner as that in Example 1. As a result, it was confirmed that dimethyl terephthalate with a purity of 99% by mass or more was obtained. The analysis result by ¹H NMR is shown in Fig. 3, and the analysis result by gas chromatography is shown in Fig. 4.

### <Decomposition of polyester fibers in colored fibers containing polyester fibers and colorant>

### [Example 4]

Into a 100-mL screw vial having a flat bottom surface, sodium methoxide (0.29 g, 5.2 mmol, 2.9% by mass relative to the mass of polyester fibers described later) was added. Furthermore, methanol (4 mL, 31.6% by mass relative to the mass of polyester fibers described later) and dimethyl carbonate (30 mL, 321% by mass relative to the mass of polyester fibers described later) were added thereto. These blended components were uniformly dissolved to prepare a catalyst composition. Colored clothing (the ratio of polyester fibers in all fibers: 100% by mass, including a dye of red, black, blue, or the like) were cut into a size of about 2 cm × 2 cm. A plurality of these cut pieces (10 g) was added to the above obtained catalyst composition in the screw vial. The mixture of the catalyst composition and the cut pieces was stirred at 70°C for 1 hour while rotating the stirring bar in the screw vial using a magnetic stirrer and an oil bath to decompose the polyester fibers. The contents in the screw vial were then filtered during hot state while maintaining the temperature at 70°C without cooling. Methanol (100 mL) was added to the filtrate, and the resulting white crystals were filtered off, washed with water (50 mL), and dried to obtain dimethyl terephthalate (7.4 g).

The resulting dimethyl terephthalate was analyzed by ¹H NMR and gas chromatography in the same manner as that in Example 1, and similar data to those in Example 1 was obtained. Thus, it was confirmed that dimethyl terephthalate with a purity of 99% by mass or more was obtained.

Furthermore, the total nitrogen content of the resulting dimethyl terephthalate was determined quantitatively in the same manner as that in Example 3, and the content of the nitrogen components of dimethyl terephthalate was 19 ppm.

### <Decomposition of polyester fibers in mixed fibers containing polyester fibers, rayon and polyurethane fibers>

### [Example 5]

Mixed fibers (the ratio of polyester fibers in all fibers: 54% by mass, the ratio of rayon: 39% by mass, the ratio of polyurethane fibers: 7% by mass) were cut to a size of about 2 cm × 2 cm. Dimethyl terephthalate (3.4 g) was obtained by decomposing polyester fibers in the cut pieces in the same manner as that in Example 4, except that a plurality of cut pieces (10 g) of the mixed fibers was used instead of the above-mentioned plurality of cut pieces (10 g) of the colored clothing. In the present Example, the use amount of methanol at the time of the decomposition was 58.6% by mass relative to the amount of polyester fibers in the mixed fibers.

The resulting dimethyl terephthalate was analyzed by ¹H NMR and gas chromatography in the same manner as that in Example 1, and similar data to those in Example 1 was obtained. Thus, it was confirmed that dimethyl terephthalate with a purity of 99% by mass or more was obtained.

### <Decomposition of polyester in a single-layer film containing polyester and component other than polyester>

### [Example 6]

Individual packaging films of confectionery were washed with water and the dried composite films (single-layer films containing a polyester and a component other than polyesters) were cut into a size of about 2 cm × 2 cm. Dimethyl terephthalate (3.4 g) was obtained by decomposing polyester in the cut pieces in the same manner as that in Example 4, except that the plurality of cut pieces (10 g) of the single-layer film was used instead of the above-described plurality of cut pieces (10 g) of the colored clothing.

The resulting dimethyl terephthalate was analyzed by ¹H NMR and gas chromatography in the same manner as that in Example 1, and similar data to those in Example 1 was obtained. Thus, it was confirmed that dimethyl terephthalate with a purity of 99% by mass or more was obtained.

### <Decomposition of polyester fibers in colored fibers containing polyester fibers and colorant>

### [Comparative Example 1]

Dimethyl terephthalate (2.1 g) was obtained by decomposing the polyester fibers in the same manner as that in Example 4, except that methanol (34 mL) was used without using dimethyl carbonate instead of methanol (4 mL) and dimethyl carbonate (30 mL).

The resulting dimethyl terephthalate was analyzed by ¹H NMR in the same manner as that in Example 4, but unlike the result in Example 4, a signal presumed to be an oligomeric impurity was detected. That is, the resulting dimethyl terephthalate had a low purity.

### [Comparative Example 2]

Dimethyl terephthalate (4.6 g) was obtained by decomposing polyester fibers in the same manner as that in Example 4, except that toluene (manufactured by Kishida Chemical Co., Ltd., 30 mL) was used instead of dimethyl carbonate (30 mL).

The resulting dimethyl terephthalate was analyzed by ¹H NMR in the same manner as that in Example 4, but unlike the result in Example 4, a signal presumed to be an oligomeric impurity was detected. That is, the resulting dimethyl terephthalate had a low purity.

### [Comparative Example 3]

Dimethyl terephthalate (4.0 g) was obtained by decomposing polyester fibers in the same manner as that in Example 4, except that dimethyl sulfoxide (manufactured by Kishida Chemical Co., Ltd., 30 mL) was used instead of dimethyl carbonate (30 mL).

The resulting dimethyl terephthalate was analyzed by ¹H NMR in the same manner as that in Example 4, but unlike the result in Example 4, a signal presumed to be an oligomeric impurity was detected. That is, the resulting dimethyl terephthalate had a low purity.

### [Comparative Example 4]

Into a 300-mL eggplant flask, sodium hydroxide (NaOH) (manufactured by FUJIFILM Wako Pure Chemical Corporation, 50 mg, 1.25 mmol, 0.25% by mass relative to the mass of polyester fibers described later) was added. Furthermore, ethylene glycol (manufactured by FUJIFILM Wako Pure Chemical Corporation) (100 mL) was added thereto. These blended components were uniformly dissolved to prepare a catalyst composition. Colored clothing (the ratio of polyester fibers in all fibers: 100% by mass, including a dye of red, black, blue, or the like) was cut into a size of about 2 cm × 2 cm and a plurality of these cut pieces (20 g) was added to the catalyst composition obtained above in the eggplant flask. The mixture of the catalyst composition and the cut pieces was stirred at 195°C for 5 hours while rotating a stirring bar in the eggplant flask using a magnetic stirrer and an oil bath, to decompose the polyester fibers. The reaction products in the eggplant flask were then allowed to cool to 100°C and the precipitated solids were filtered off by hot filtration, washed with water (100 mL) and dried to obtain bis(2-hydroxyethyl) terephthalate (20 g) as the decomposition product.

The total nitrogen content of the resulting bis(2-hydroxyethyl) terephthalate was determined quantitatively in the same manner as that in Example 3, and the content of the nitrogen components of bis(2-hydroxyethyl) terephthalate was 140 ppm.

The resulting bis(2-hydroxyethyl) terephthalate was noticeably more colored than dimethyl terephthalate obtained in Examples 1 to 6.

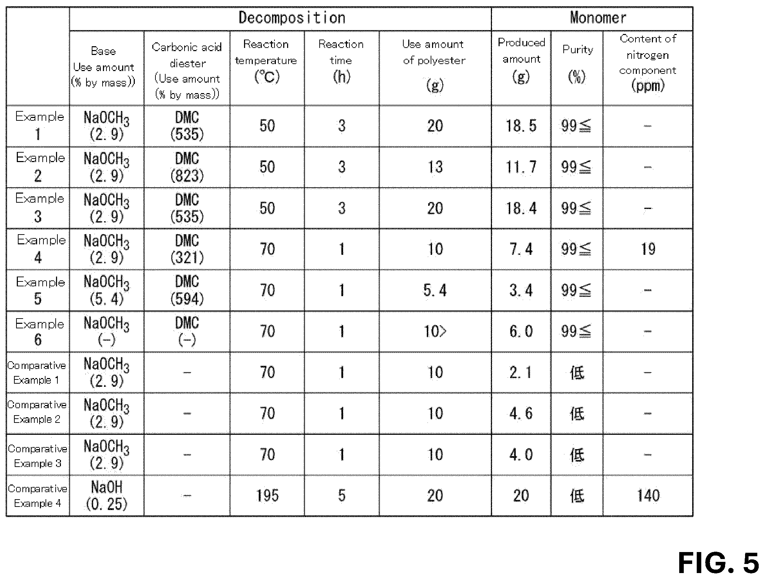

The results of Examples 1 to 6 and Comparative Examples 1 to 4 are summarized in Fig. 5. In this figure, "base (use amount (% by mass)" means "a ratio of the use amount (parts by mass) of base to the use amount (parts by mass) of polyester in the polyester-containing material". Similarly, "carbonic acid diester (use amount (% by mass) " means "a ratio of the use amount (parts by mass) of carbonic acid diester to the amount (parts by mass) of polyester in the polyester-containing material".

As is apparent from these results, in Examples 1 to 6, it was possible to decompose the polyester in the polyester-containing material at a low temperature of 50 to 70°C as the reaction temperature in a simple process without requiring additional complicated steps. As a result, a high-purity dicarboxylic acid diester (monomer) was obtained.

On the other hand, in Comparative Examples 1 to 3, high-purity dicarboxylic acid diesters (monomers) were not obtained by decomposition of the polyester in the polyester-containing material. In Comparative Examples 1 to 3, the carbonic acid diester was not used at the time of the decomposition.

In Comparative Example 4, the polyester was decomposed by a typical conventional method, but the reaction temperature was too high, and the content of the nitrogen component of the dicarboxylic acid diester (monomer) was high, so that a high-purity dicarboxylic acid diester could not be obtained.

From the comparison between Example 4 and Comparative Examples 1 to 3, it was confirmed that the use of the carbonic acid diester promoted the polyester decomposition and significantly increased the amount of the dicarboxylic acid diester produced.

### [Examples 7 to 9]

A polybutylene terephthalate (PBT) powder (100 mg) as a polyester-containing material was decomposed by carrying out the same operations as those in Example 1 (reaction temperature: 50°C, reaction time: 5 hours) using potassium methoxide (KOMe) as a base, methanol (MeOH) as a monohydric alcohol, and dimethyl carbonate (DMC) as a carbonic acid diester. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula. The addition amount of methanol is different for each example.

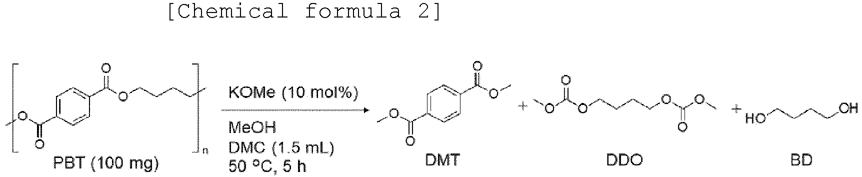

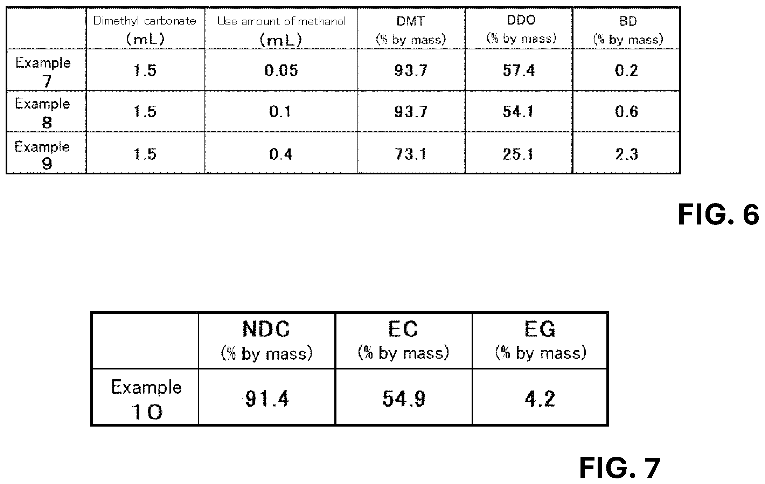

These results are shown in Fig. 6. This figure demonstrated that, in accordance with the present invention, polybutylene terephthalate could be decomposed similarly to polyethylene terephthalate.

### [Example 10]

A polyethylene naphthalate (PEN) powder (100 mg) as a polyester-containing material was decomposed by carrying out the same operations as those in Example 1 (reaction temperature: 50°C, reaction time: 1 hour) using sodium methoxide (NaOMe) as a base, methanol (MeOH) as a monohydric alcohol, and dimethyl carbonate (DMC) as a carbonic acid diester,. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula.

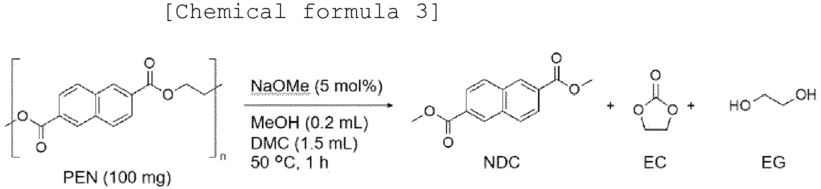

These results are shown in Fig. 7. This figure demonstrated that, in accordance with the present invention polyethylene naphthalene dicarboxylate could be decomposed similarly to polyethylene terephthalate.

### [Examples 11 to 17]

A polyethylene terephthalate powder as a polyester-containing material was decomposed by carrying out the same operations as those in Example 1, while the type of a catalyst (base), the reaction temperature, and the reaction time were changed from those in Example 1. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula.

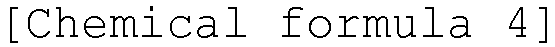

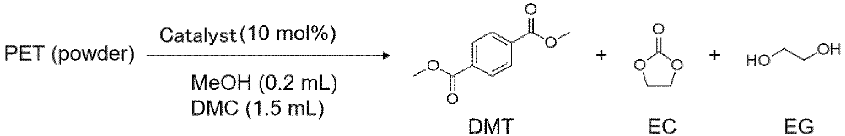

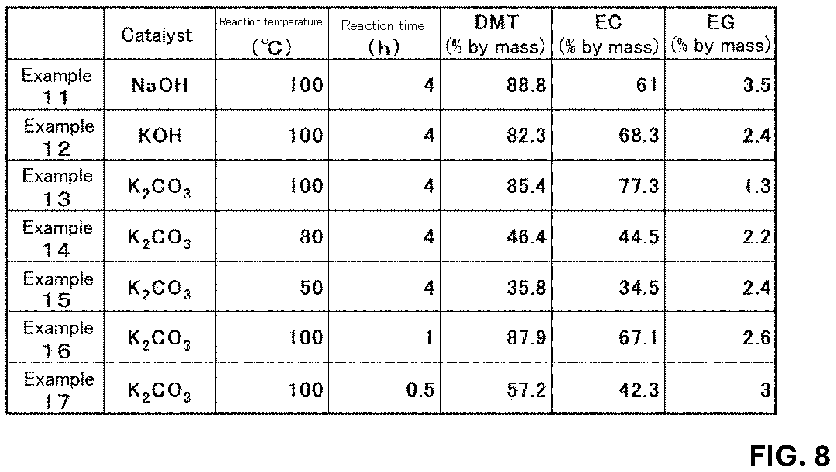

These results are shown in Fig. 8. This figure demonstrated that polyethylene terephthalate could be decomposed even when the type of the base, the reaction temperature, and the reaction time were changed.

### [Examples 18 to 20]

A polyethylene terephthalate powder (100 mg) was decomposed by carrying out the same operations as those in Example 1 (reaction temperature: 100°C, reaction time: 1 hour) using K₂CO₃ as a catalyst (base) with the amount of the catalyst changed. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula.

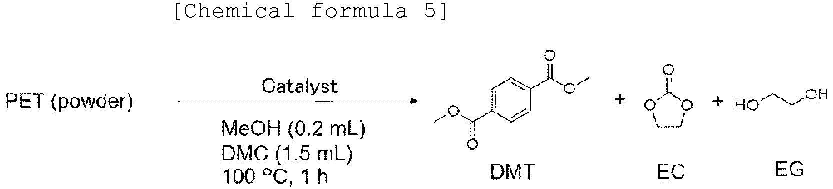

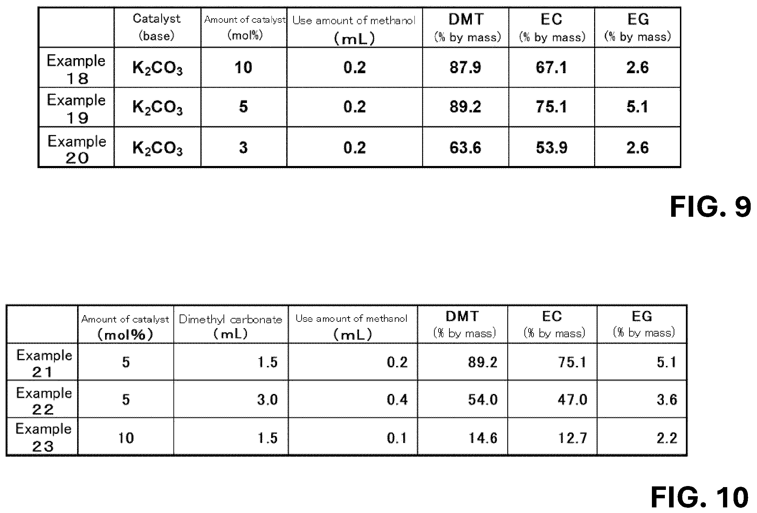

These results are shown in Fig. 9. This figure demonstrated that the polyethylene terephthalate powder could be sufficiently decomposed even when K₂CO₃ was used as a catalyst (base) .

### [Examples 21 to 23]

The polyethylene terephthalate powder (100 mg) was decomposed by carrying out the same operations as those in Example 1 (reaction temperature: 100°C, reaction time: 1 hour) except that the amounts of methanol as the solvent and dimethyl carbonate were changed from those in Example 1. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula.

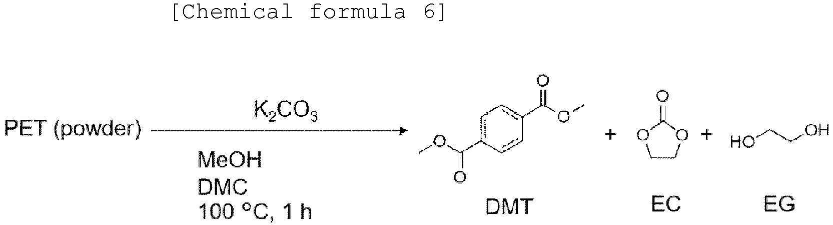

These results are shown in Fig. 10. This figure demonstrated that the polyethylene terephthalate powder could be sufficiently decomposed even when the amounts of methanol as a solvent and dimethyl carbonate were changed.

### [Examples 24 to 25]

A polyethylene terephthalate powder (100 mg) was decomposed by carrying out the same operations as those in Example 1 (reaction temperature: 100°C, reaction time: 1 hour) while water was added thereto. After the reaction, the resulting solution was filtered, and biphenyl was added thereto as an internal standard. Then, the solution was analyzed using gas chromatography ("GC-2000" manufactured by Shimadzu Corporation) to quantify the products. This reaction is represented by the following chemical reaction formula.

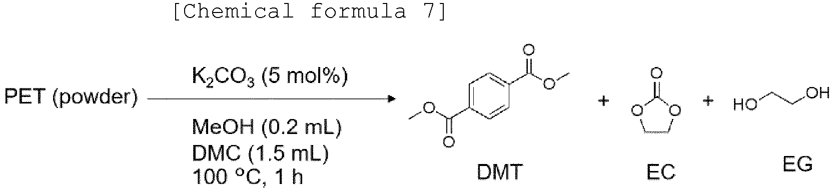

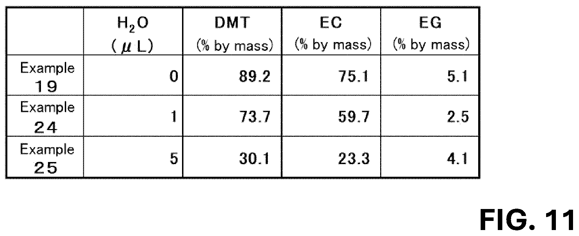

These results are shown in Fig. 11 along with the results of Example 19. This figure demonstrated that polyethylene terephthalate became hardly decomposed as the water content increased.

### [Examples 26 to 32]

A polyethylene terephthalate powder (100 mg) was decomposed by carrying out the same operations as those in Example 1 (reaction temperature: 100°C, reaction time: 0.5 hours) while a solvent other than methanol and dimethyl carbonate was added thereto. After the reaction, the resulting solution was filtered, and biphenyl was added thereto as an internal standard. Then, the solution was analyzed using gas chromatography ("GC-2000" manufactured by Shimadzu Corporation) to quantify the products.

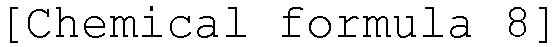

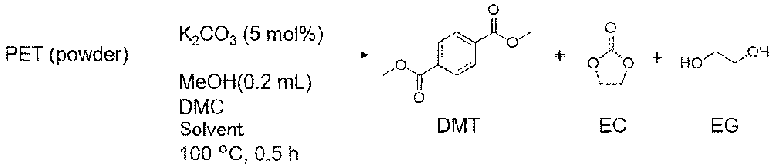

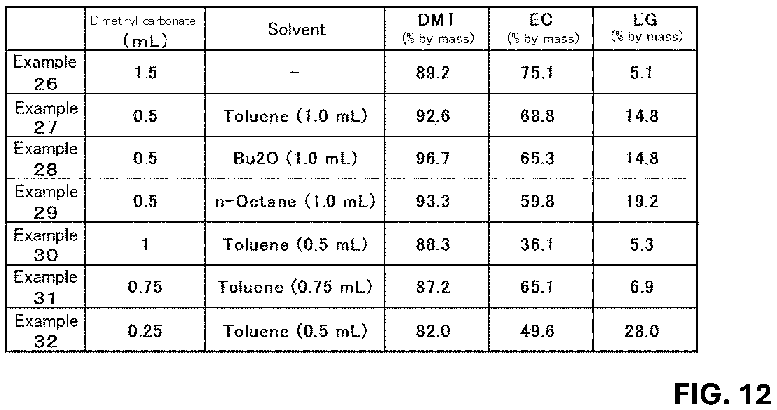

The results are shown in Fig. 12. This figure demonstrated that the polyethylene terephthalate powder could be decomposed even when a solvent other than methanol and dimethyl carbonate was added.

### [Example 33]

Cut pieces (1 g) of a white coat for testing (the ratio of polyester fibers in all fibers: 100% by mass) was depolymerized by carrying out the same operations as those in Example 1 (reaction temperature: 100°C, reaction time: 3 hours) while K₂CO₃ was used as a catalyst (base) and the amount of the catalyst was changed to 7.4% by mass relative to the mass of the polyester. After completion of the reaction, dimethyl carbonate and methanol were recovered by concentrating the reaction solution, and then the residue was distilled under reduced pressure by a Kugelrohr distillation apparatus to obtain dimethyl terephthalate (0.90 g). The purity of the resulting dimethyl terephthalate was equal to or more than 99% determined by ¹H NMR and gas chromatography.

### [Example 34]

Cut pieces (1 g) of colored clothing (the ratio of polyester fibers in all fibers: 100% by mass, including a dye of red, black, blue, or the like) was depolymerized under the conditions of Example 33 instead of the white coat for testing (the ratio of polyester fibers in all fibers: 100% by mass). After completion of the reaction, dimethyl carbonate and methanol were recovered by concentrating the reaction solution, and then the residue was distilled under reduced pressure by a Kugelrohr roll distillation apparatus to obtain dimethyl terephthalate (0.87 g). The purity of the resulting dimethyl terephthalate was equal to or more than 99% determined by ¹H NMR and gas chromatography.

### [Example 35]

Cut pieces (1 g) of a white coat for chemical experiments (the ratio of polyester fibers in all fibers: 65% by mass, the ratio of cotton: 35% by mass) was depolymerized under the conditions of Example 33 instead of the white coat for testing (the ratio of polyester fibers in all fibers: 100% by mass). After completion of the reaction, dimethyl carbonate and methanol were recovered by concentrating the reaction solution, and then the residue was distilled under reduced pressure by a Kugelrohr roll distillation apparatus to obtain dimethyl terephthalate (0.52 g). The purity of the resulting dimethyl terephthalate was equal to or more than 99% determined by ¹H NMR and gas chromatography.

### (Embodiment 2)

In Embodiment 1, the apparatus used for the polyester decomposition is not particularly limited, but in the present embodiment, a method for decomposing the polyester using a mechanochemical reaction device will be described.

### <<Decomposition method of polyester>>

The decomposition method of a polyester according to the present embodiment includes a step of depolymerizing (decomposing) a polyester contained in a material containing a polyester (polyester-containing material) by blending (mixing) a base, a monohydric alcohol, a carbonic acid diester, and a polyester-containing material, and using a mechanochemical reaction device. Then, the polyester-containing material may be one type or two or more types selected from the group consisting of a material containing fibers of a polyester (polyester fibers), a film containing a polyester and a component other than polyesters, and a lump containing a polyester and a component other than polyesters.

According to the decomposition method of the present embodiment, the polyester contained in the polyester-containing material can be decomposed without requiring a high temperature, a halogenated solvent, an excess amount of an organic solvent, and an excess amount of a base. Moreover, a dicarboxylic acid diester, which is a monomer, can be obtained as a main depolymerization product (decomposition product) with high purity and good yield without requiring additional complicated steps after the decomposition. That is, according to the decomposition method of the present embodiment, the polyester contained in the polyester-containing material can be efficiently decomposed in a process having a small environmental load, and so the dicarboxylic acid diester, which is a monomer, can be obtained with high purity and good yield. Furthermore, a polyester-containing material, of which the polyester has conventionally been difficult to decompose at relatively low temperatures unlike materials such as PET film, can be used.

Incidentally, the polyester-containing material, base, monohydric alcohol, carbonate diester, solvent, and other components that can be used in the present embodiment are the same as those in the first embodiment.

### <Mechanochemical reaction device>

Mechanochemical reaction devices used in the polyester decomposition are devices for carrying out well-known mechanochemical reactions, i.e., for applying a mechanical stress (external force) to materials to cause chemical changes with the surrounding materials.

However, in the present embodiment, it is not essential to cause a mechanochemical reaction during polyester decomposition. In the present embodiment, the materials that fall within the respective specific ranges as described above are used as the polyester-containing materials. However, use of such a mechanochemical reaction device at the time of decomposition can easily decompose the polyester even when the above-mentioned polyester-containing materials, of which the polyester has conventionally been difficult to decompose at relatively low temperatures, are used.

The mechanochemical reaction device used in the polyester decomposition is not particularly limited as long as the mechanochemical reaction can be carried out, and may be a known device. Examples of the mechanochemical reaction device include a device including a container and a stress applying unit (external force applying unit) for applying a mechanical stress to the object, with the container being for accommodating an object to be subjected to a mechanochemical reaction and the stress applying unit.

Examples of the stress applying unit include a solid made of a hard material such as a metal and capable of applying impact or shear to the object to be subjected to a mechanochemical reaction. Examples of the stress applying unit include those having a ball shape or a bead shape.

Examples of the material for the container and the stress applying unit that constitute the mechanochemical reaction device include stainless steel (SUS), zirconium oxide (also known as zirconia), polytetrafluoroethylene, tungsten carbide, and agate.

The mechanochemical reaction device is not particularly limited, and may be, for example, a batch type device or a continuous type device. More specific examples of the mechanochemical reaction device include a ball mill, a bead mill, a planetary mill, and a twin-screw kneading extruder. In particular, the mechanochemical reaction device is preferably a ball mill in terms of high versatility and allowing the polyester decomposition to proceed more easily.

### <Reaction condition>

The decomposition of the polyesters in the polyester-containing material can be carried out by blending a base, a monohydric alcohol, a carbonic acid diester, a polyester-containing material, a solvent, if necessary, and other components, if necessary, and reacting the resulting blend using a mechanochemical reaction device.

The blending order of these raw materials is not particularly limited. For example, the raw materials may be separately blended in any order. Furthermore, the raw materials may be blended by first preparing a raw material composition which is a blend of a base, a monohydric alcohol, a carbonic acid diester, a solvent, if necessary, and other components, if necessary (for example, a blend of all raw materials other than the polyester-containing material), and then mixing the resulting raw material composition with the polyester-containing material. In this case, as will be described later, the blend amount of the base may be a catalytic amount (the base may serve as a catalyst) (the raw material composition may be a catalyst composition).

The reaction temperature during polyester decomposition can be appropriately adjusted in consideration of the types of the raw materials to be used and the like, and the decomposition may be carried out at room temperature (normal temperature) or under heating conditions. In the present embodiment, the polyester decomposition proceeds sufficiently even at room temperature, but may proceed faster under heating conditions. When the polyester is decomposed at room temperature (normal temperature), the reaction temperature may be any of 15 to 25°C, 15 to 20°C, and 20 to 25°C.

When the polyester is decomposed under heating conditions, the reaction temperature (reaction temperature in the decomposition step) is not particularly limited as long as it is 30°C to 150°C, but is more preferably 30°C to 100°C. When the reaction temperature under the heating conditions is equal to or higher than these lower limit values, the effect obtained by the heating becomes higher. When the reaction temperature under the heating conditions is equal to or lower than these upper limit values, excessive heating is suppressed.

According to the present embodiment, as described above, the polyester can be decomposed at a low temperature such as 100°C or lower in spite of the use of a polyester-containing material of which the polyester has conventionally been difficult to decompose at relatively low temperatures unlike materials such as PET film. As a result, unlike a case where the decomposition is carried out at a high temperature (for example, 300°C or higher), the amount of by-products produced during polyester decomposition can be reduced, and the coloring of the dicarboxylic acid diester (monomer), which is a main decomposition product, can also be reduced.

The polyester may be decomposed under any of atmospheric pressure, reduced pressure, and pressurization. The polyester may be decomposed either under an atmosphere or an inert gas atmosphere.

Furthermore, the reaction time at the time of the polyester decomposition can be appropriately adjusted in consideration of other reaction conditions such as the reaction temperature, and is not particularly limited. The reaction time for the polyester decomposition is preferably 3 to 60 minutes, more preferably 3 to 40 minutes, and still more preferably 3 to 25 minutes.

In the present embodiment, for example, it can be determined that the time when the polyester disappears is the end time of the polyester decomposition. Therefore, the time required for the polyester to disappear can be regarded as the reaction time for the polyester decomposition. The time required for the polyester to disappear can also be determined, for example, by the time required for the mass reduction of the polyester-containing material to cease.

In the blend immediately after blending the respective raw materials, the unreacted polyester-containing material does not dissolve and remains undissolved in the other liquid components. In a blend (reactants) in which the polyester decomposition is in progress, both the unreacted polyester-containing material and the polyester-containing material during the reaction of the polyester or after the reaction typically do not dissolve, and so they remain undissolved in the other liquid components. On the other hand, glycols and dicarboxylic acid diesters, which are reaction products of the polyester decomposition, are typically dissolved in the liquid component.

The blend amount of the polyester-containing material is preferably 5 to 35 g per 1 L of the container volume constituting the mechanochemical reaction device, and may be, for example, any of 5 to 25 g and 5 to 15 g, any of 8 to 35 g and 15 to 35 g, or 8 to 25 g. When the blend amount of the polyester-containing material falls within such a range, the polyester can be decomposed more efficiently. Here, for example, when the mechanochemical reaction device is a ball mill, the container is a grinding jar.

The number of the stress applying unit constituting the mechanochemical reaction device is 1 or 2 or more, and can be arbitrarily selected depending on the type of the stress applying unit, and is not particularly limited. For example, when the mechanochemical reaction device is a ball mill or a planetary mill, the stress applying unit is a ball (grinding ball). When the mechanochemical reaction device is a bead mill, the stress applying unit is a bead. Here, the number of balls or beads can be appropriately adjusted depending on the volume of the container constituting the mechanochemical reaction device. For example, the number of balls or beads is preferably such that their total volume of the balls or beads accounts for 3 to 30% of the volume of the container, and may be, for example, any number falling within any of 3 to 20% and 3 to 10%.

If the mechanochemical reaction device is a twin-screw kneading extruder, the stress applying unit is a screw, and the number thereof is 2.

When the mechanochemical reaction device is a ball mill or a planetary mill, the total volume of the stress applying units constituting the mechanochemical reaction device is preferably 1,000 to 7,000 mm³ relative to 1 g of the blend amount of the polyester-containing material, and may be, for example, any of 1,000 to 5,500 mm³ and 1,000 to 4,000 mm³, any of 3, 000 to 7,000 mm³ and 5, 500 to 7,000 mm³, or 3, 000 to 5, 500 mm³. Here, the total volume of the stress applying units is the total value of the volumes of all the stress applying units.

When the mechanochemical reaction device is a ball mill, the frequency at which it is actuated to stir the blend is preferably 15 to 40 Hz, and may be, for example, any of 15 to 27 Hz and 15 to 23 Hz, any of 23 to 40 Hz and 28 to 40 Hz, or 23 to 27 Hz.

### <Post-processing condition and taking-out condition>

After the end of the above-described step of decomposing a polyester, post-processing is carried out by a known method, so that a dicarboxylic acid diester, which is one main decomposition product, can be taken out with high purity. For example, a high-purity dicarboxylic acid diester can be obtained by decomposing a polyester, followed by filtration of the resulting reaction products, distillation (condensation) of volatile matter, and washing the resulting solid matter with water, methanol, or water and methanol. The resulting dicarboxylic acid diester may be purified by crystallization, distillation, or the like, if necessary.

The reaction product between the glycol as the other main decomposition product and the carboxylic acid diester can also be taken out by appropriately adjusting the post-processing conditions and the taking-out conditions similarly to in the case of the dicarboxylic acid diester.

According to the present embodiment, decomposition of polyesters is possible at a low temperature of, for example, 100°C or lower as previously explained. Therefore, the impurity content and coloring of the dicarboxylic acid diester can be reduced, unlike a case where the decomposition of polyesters is carried out at a high temperature. Thus, a high-purity dicarboxylic acid diester (monomer) in which the coloring is suppressed is obtained in a simplified step of washing with water, methanol, and the like as described above without requiring additional complicated steps. In addition, even if the polyester-containing material originally contains a colorant, the coloring of the dicarboxylic acid diester (monomer) derived from the colorant can also be reduced in a simplified step of washing with water, methanol, and the like as described above.

On the contrary, when decomposition of polyesters is carried out at a high temperature by a known method, a complicated step including decoloring with a high boiling point solvent and decomposition of a dye with an oxidant is necessary in order to obtain high-purity monomers.

It is needless to say that by using the polyester production method according to Embodiment 1, the polyester can be produced using the polyester decomposition products (dicarboxylic acid diester, glycol) obtained by the polyester decomposition method according to the present embodiment.

Hereinafter, the invention according to the present embodiment will be described in more detail with reference to specific examples. Polyethylene terephthalate (PET) was used as the polyester. However, the invention according to the present embodiment is not limited to the following examples.

### <<Decomposition of polyester>>

### <Decomposition of polyester fibers>

### [Example 36]

A white coat for testing (the ratio of polyester fibers in all fibers: 100% by mass) was cut into a size of about 2 cm × 2 cm. A plurality of these cut pieces (0.1 g) was charged into the inside of a stainless-steel grinding jar with a volume of 10 mL.

Here, the ratio (% by mass) of polyester fibers in all fibers has the same meaning as the above-described ratio of the content (parts by mass) of polyester to the total mass (parts by mass) of the polyester-containing material in the polyester-containing material. This applies to subsequent other Examples and Comparative Examples.

Furthermore, sodium methoxide (NaOCH₃) (manufactured by FUJIFILM Wako Pure Chemical Corporation, the same applies below, 1.4 mg, 0.026 mmol, 1.4% by mass relative to the mass of polyester fibers), methanol (CH₃OH) (manufactured by Kishida Chemical Co., Ltd., the same applies below, 0.02 mL, 15.8% by mass relative to the mass of polyester fibers), dimethyl carbonate (DMC) (manufactured by Tokyo Chemical Industry Co., Ltd., the same applies below, 0.2 mL, 214% by mass relative to the mass of polyester fibers), and stainless steel (SUS) grinding balls (diameter: 10 mm, 1 piece) were charged into the inside of the grinding jar, and the lid was closed to hermetically seal the blend. This grinding jar was installed and fixed in a ball mill reaction device ("MM400" manufactured by Retsch GmbH., the same applies below), and the blend was stirred at room temperature at a frequency of 25 Hz for 10 minutes to decompose the polyester fibers. Next, dimethyl carbonate (10 mL) was added to the reaction products inside the grinding jar, so that the deposited object was dissolved. The resulting liquid substance was filtered to obtain a solution of dimethyl terephthalate.

Then, biphenyl was added to the resulting solution as an internal standard. The solution was analyzed using gas chromatography ("GC-2000" manufactured by Shimadzu Corporation), and as a result, it was confirmed that the products were dimethyl terephthalate and ethylene carbonate. Quantifying the products, the yields of dimethyl terephthalate and ethylene carbonate were 88% and 73%, respectively.

### [Example 37]

Dimethyl terephthalate (yield: 88%) and ethylene carbonate (yield: 72%) were obtained in the same manner as that in Example 36, except that potassium methoxide (KOCH₃) (manufactured by FUJIFILM Wako Pure Chemical Corporation, 1.8 mg, 0.026 mmol, 1.8% by mass relative to the mass of polyester fibers) was used instead of sodium methoxide.

### [Example 38]

Dimethyl terephthalate (yield: 54%) and ethylene carbonate (yield: 42%) were obtained in the same manner as that in Example 36, except that the use amount of methanol was changed to 0.01 mL instead of 0.02 mL.

### [Example 39]

Dimethyl terephthalate (yield: 62%) and ethylene carbonate (yield: 49%) were obtained in the same manner as that in Example 36, except that the use amount of methanol was changed to 0.03 mL instead of 0.02 mL.

### [Example 40]

Dimethyl terephthalate (yield: 62%) and ethylene carbonate (yield: 47%) were obtained in the same manner as that in Example 36, except that the use amount of methanol was changed to 0.04 mL instead of 0.02 mL.

### [Example 41]

Dimethyl terephthalate (yield: 77%) and ethylene carbonate (yield: 64%) were obtained in the same manner as that in Example 36, except that the use amount of dimethyl carbonate at the time of the decomposition was changed to 0.1 mL instead of 0.2 mL.

### [Example 42]

Dimethyl terephthalate (yield: 79%) and ethylene carbonate (yield: 62%) were obtained in the same manner as that in Example 36, except that the use amount of dimethyl carbonate at the time of the decomposition was changed to 0.4 mL instead of 0.2 mL.

### [Example 43]

Dimethyl terephthalate (yield: 87%) and ethylene carbonate (yield: 72%) were obtained in the same manner as that in Example 36, except that ten stainless steel grinding balls with a diameter of 5 mm were used instead of one stainless steel grinding ball with a diameter of 10 mm.

### [Example 44]

Dimethyl terephthalate (yield: 61%) and ethylene carbonate (yield: 49%) were obtained in the same manner as that in Example 36, except that a grinding jar made of zirconium oxide (ZrO₂) with a volume of 10 mL was used instead of the grinding jar made of stainless steel with a volume of 10 mL and that one zirconium oxide grinding ball with a diameter of 10 mm was used instead of one stainless steel grinding ball with a diameter of 10 mm.

### [Example 45]

Dimethyl terephthalate (yield: 64%) and ethylene carbonate (yield: 51%) were obtained in the same manner as that in Example 36, except that the frequency at which the blend was stirred was changed to 20 Hz instead of 25 Hz.

### [Example 46]

Dimethyl terephthalate (yield: 76%) and ethylene carbonate (yield: 62%) were obtained in the same manner as that in Example 36, except that the frequency at which the blend was stirred was changed to 30 Hz instead of 25 Hz.

### [Example 47]

Dimethyl terephthalate (yield: 52%) and ethylene carbonate (yield: 41%) were obtained in the same manner as that in Example 36, except that the stirring time of the blend was 5 minutes instead of 10 minutes.

### [Example 48]

Dimethyl terephthalate (yield: 90%) and ethylene carbonate (yield: 68%) were obtained in the same manner as that in Example 36, except that the stirring time of the blend was 15 minutes instead of 10 minutes.

### [Example 49]

Dimethyl terephthalate (yield: 95%) and ethylene carbonate (yield: 78%) were obtained in the same manner as that in Example 36, except that the stirring time of the blend was 20 minutes instead of 10 minutes.

### [Comparative Example 5]

The polyester fibers were subjected to decomposition in the same manner as that in Example 36, except that sodium methoxide was not used. However, the polyester fibers could not be decomposed, and neither dimethyl terephthalate nor ethylene carbonate was produced (yield: 0%).

### [Comparative Example 6]

Dimethyl terephthalate (yield: 10%) and ethylene carbonate (yield: 7%) were obtained in the same manner as that in Example 36, except that methanol (0.2 mL) was used without dimethyl carbonate instead of using both methanol (0.02 mL) and dimethyl carbonate (0.2 mL).

### [Comparative Example 7]

Dimethyl terephthalate (yield: 22%) was obtained in the same manner as that in Example 36, except that toluene (manufactured by Kishida Chemical Co., Ltd., 0.2 mL) was used instead of dimethyl carbonate (0.2 mL). Here, ethylene carbonate was not obtained.

### [Reference Example 1]

A white coat for testing (the ratio of polyester fibers in all fibers: 100% by mass) was cut into a size of about 2 cm × 2 cm. A plurality of these cut pieces (0.1 g) was charged into the inside of a 2-mL screw vial having a flat bottom surface.

Furthermore, sodium methoxide (1.4 mg, 0.026 mmol, 1.4% by mass relative to the mass of polyester fibers), methanol (0.02 mL, 15.8% by mass relative to the mass of polyester fibers), and dimethyl carbonate (0.2 mL, 214% by mass relative to the mass of polyester fibers) were added into the inside of the screw vial. The stirring bar was rotated inside the screw vial to stir the blend at 50°C for 10 minutes using a magnetic stirrer and an aluminum block heater to decompose the polyester fibers. Dimethyl carbonate (10 mL) was then added to the reaction products inside the screw vial, so that the deposited object was dissolved. The resulting liquid substance was filtered to obtain a solution of dimethyl terephthalate.

The resulting solution was analyzed in the same manner as that in Example 36, and the yield of dimethyl terephthalate was found to be 23% and the yield of ethylene carbonate was found to be 32%.

### [Example 50]

A white coat for testing (the ratio of polyester fibers in all fibers: 100% by mass) was cut into a size of about 2 cm × 2 cm. A plurality of these cut pieces (1 g) was charged into the inside of a stainless-steel grinding jar with a volume of 50 mL.

Furthermore, sodium methoxide (14 mg, 0.5 mmol, 1.4% by mass relative to the mass of polyester fibers), methanol (0.2 mL, 15.8% by mass relative to the mass of polyester fibers), dimethyl carbonate (1.5 mL, 160% by mass relative to the mass of polyester fibers), and stainless steel grinding balls (diameter: 10 mm, 5 pieces) were charged into the inside of the grinding jar, and the lid was closed to hermetically seal the blend. This grinding jar was installed and fixed in a ball mill reaction device, and the blend was stirred at room temperature at a frequency of 25 Hz for 15 minutes to decompose the polyester fibers. Next, dimethyl carbonate (30 mL) was added to the reaction products inside the grinding jar, so that the deposited object was dissolved. The resulting liquid substance was filtered to obtain a solution of dimethyl terephthalate as a filtrate.
The filtrate was concentrated under reduced pressure, and the residue was washed with water (20 mL) and methanol (10 mL), and dried to obtain dimethyl terephthalate (yielded amount: 0.90 g, yield: 86%).

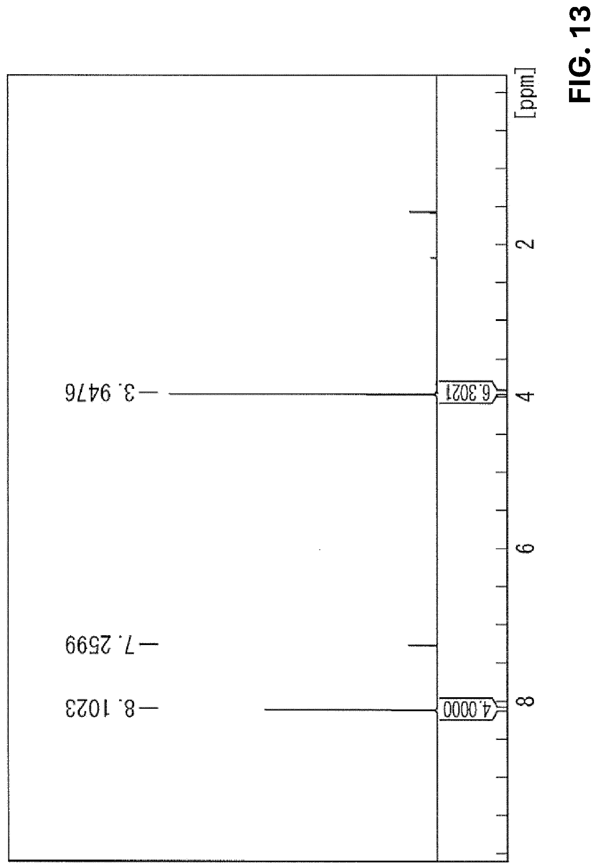

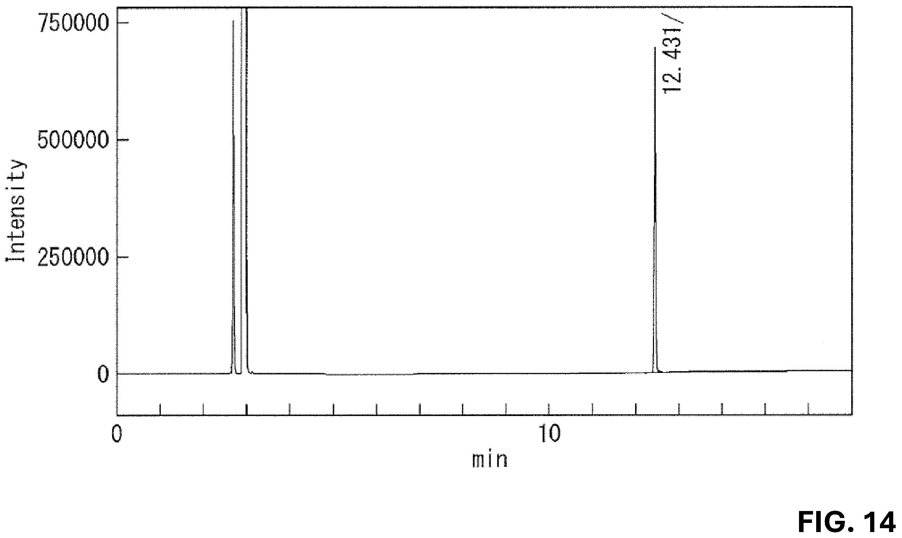

The resulting dimethyl terephthalate was analyzed by ¹H NMR (device: "Bruker Avance III (registered trademark)" 600 MHz manufactured by BRUKER Co., the same applies below) and gas chromatography (device: "GC-2000" manufactured by Shimadzu Corporation, the same applies below). As a result, it was confirmed that dimethyl terephthalate with a purity of 99% by mass or more was obtained. The analysis result by ¹H NMR is shown in Fig. 13, and the analysis result by gas chromatography is shown in Fig. 14.

### <Decomposition of polyester fibers in mixed fibers containing polyester fibers and cotton>

### [Example 51]

A white coat for chemical experiments (the ratio of polyester fibers in all fibers: 65% by mass, the ratio of cotton: 35% by mass) was cut into a size of about 2 cm × 2 cm.

Here, the ratio (% by mass) of cotton in all fibers refers to the ratio of the content (parts by mass) of cotton to the total mass (parts by mass) of the polyester-containing material in the polyester-containing material. This applies to fibers other than cotton in subsequent other Examples.

Next, dimethyl terephthalate (yielded amount: 0.56 g, yield: 86%) was obtained by decomposing polyester fibers in cut pieces in the same manner as that in Example 50, except that a plurality of cut pieces (1 g) of this white coat for chemical experiments was used instead of the above-described plurality of cut pieces (1 g) of the white coat for testing, and that the use amount of sodium methoxide was changed to 30 mg instead of 14 mg.

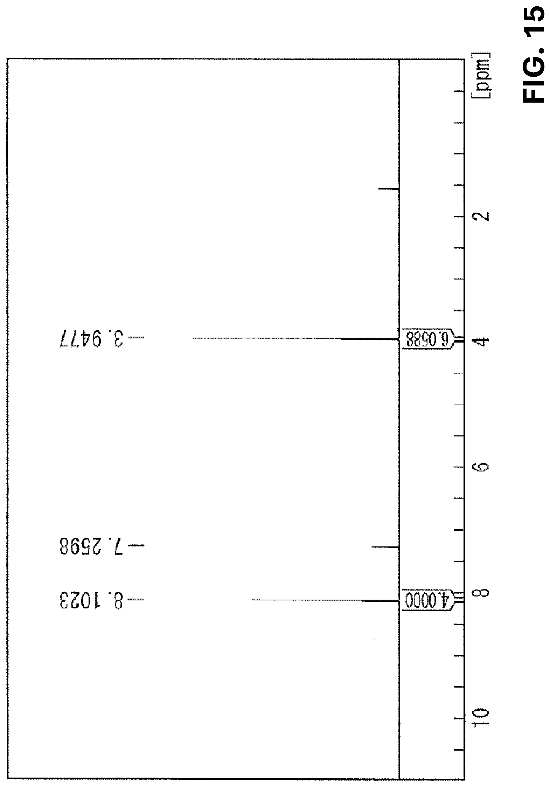

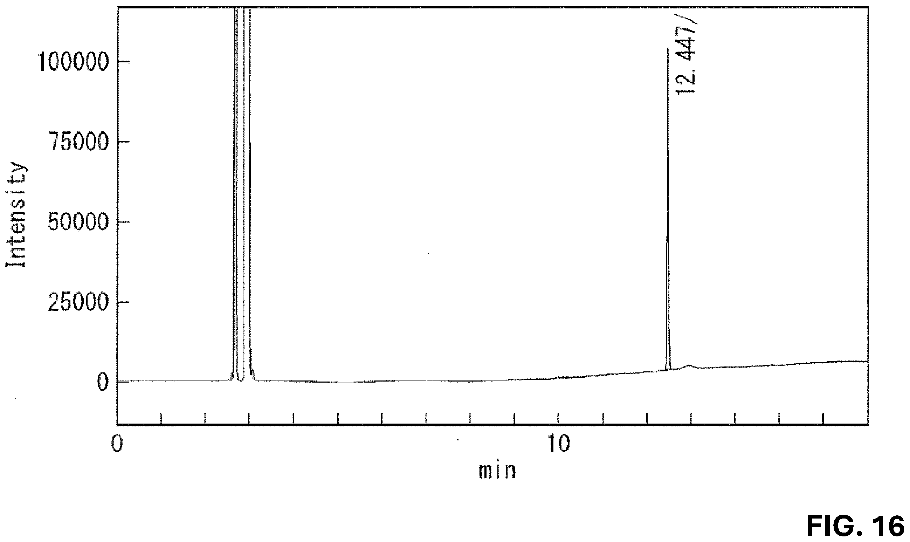

The resulting dimethyl terephthalate was analyzed by ¹H NMR and gas chromatography in the same manner as that in Example 50. As a result, it was confirmed that dimethyl terephthalate with a purity of 99% by mass or more was obtained. The analysis result by ¹H NMR is shown in Fig. 15, and the analysis result by gas chromatography is shown in Fig. 16.

### <Decomposition of polyester fibers in colored fibers containing polyester fibers and colorant>

### [Example 52]

Colored clothing (the ratio of polyester fibers in all fibers: 100% by mass, including a dye of red, black, blue, or the like) was cut into a size of about 2 cm × 2 cm. Dimethyl terephthalate (yielded amount: 0.75 g, yield: 73%) was obtained by decomposing polyester fibers in the cut pieces in the same manner as that in Example 43, except that the plurality of cut pieces (1 g) of the colored clothing was used instead of the above-described plurality of cut pieces (1 g) of the white coat for testing.

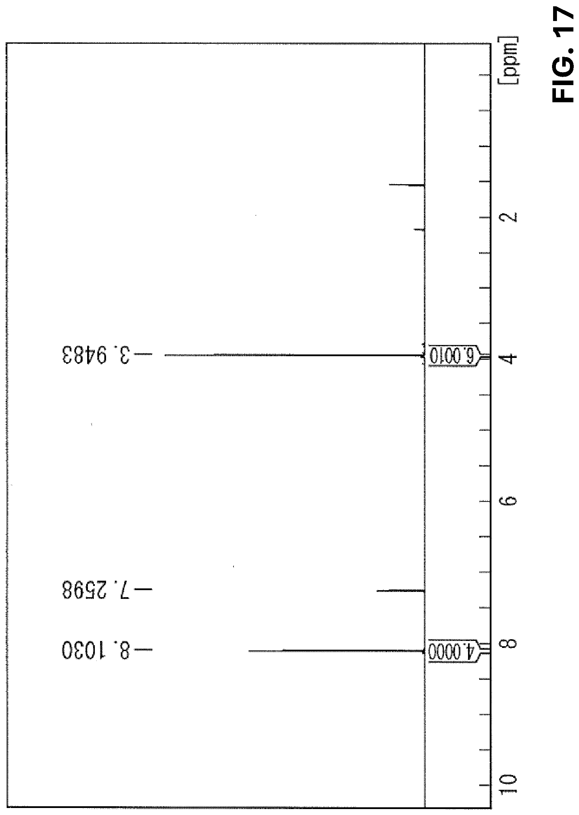

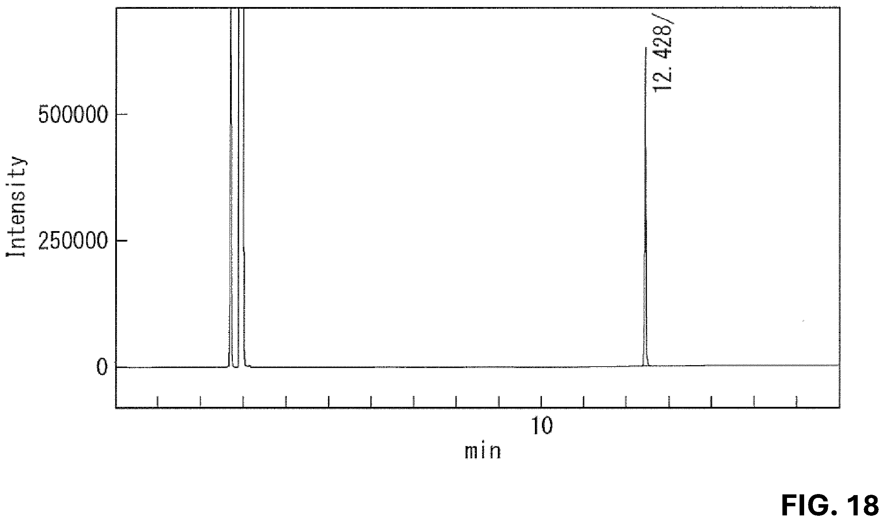

The resulting dimethyl terephthalate was analyzed by ¹H NMR and gas chromatography in the same manner as that in Example 50. As a result, it was confirmed that dimethyl terephthalate with a purity of 99% by mass or more was obtained. The analysis result by ¹H NMR is shown in Fig. 17, and the analysis result by gas chromatography is shown in Fig. 18.

### <Decomposition of polyester fibers in mixed fibers containing polyester fibers and polyurethane fibers>

### [Example 53]

Mixed fibers (the ratio of polyester fibers in all fibers: 87% by mass, the ratio of polyurethane fibers: 13% by mass) were cut to a size of about 2cm × 2cm. Dimethyl terephthalate (yielded amount: 0.62 g, yield: 70%) was obtained by decomposing polyester fibers in the cut pieces in the same manner as that in Example 50, except that a plurality of cut pieces (1 g) of the mixed fibers was used instead of the above-mentioned plurality of cut pieces (1 g) of a white coat for testing, and that the use amount of sodium methoxide was changed to 30 mg instead of 14 mg.

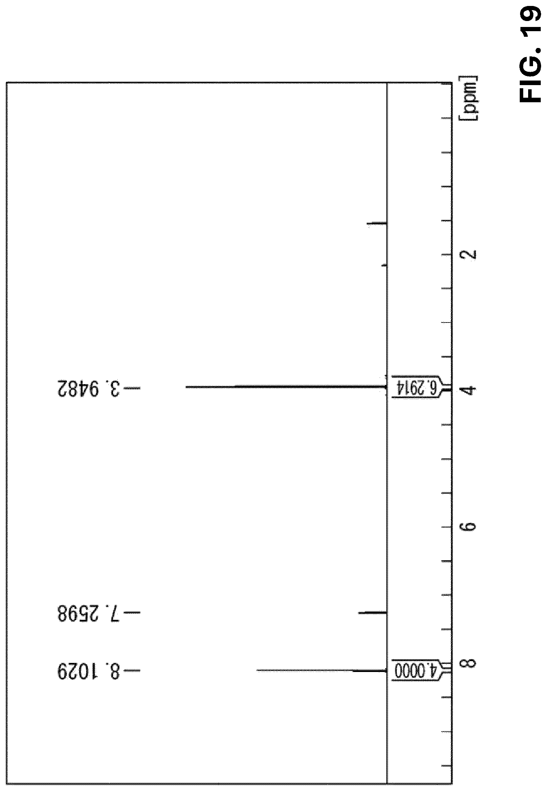

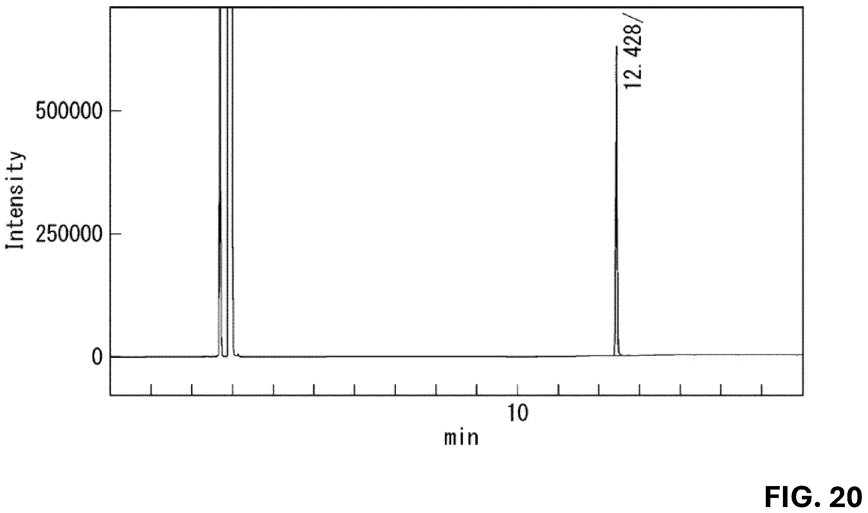

The resulting dimethyl terephthalate was analyzed by ¹H NMR and gas chromatography in the same manner as that in Example 50. As a result, it was confirmed that dimethyl terephthalate with a purity of 99% by mass or more was obtained. The analysis result by ¹H NMR is shown in Fig. 19, and the analysis result by gas chromatography is shown in Fig. 20.

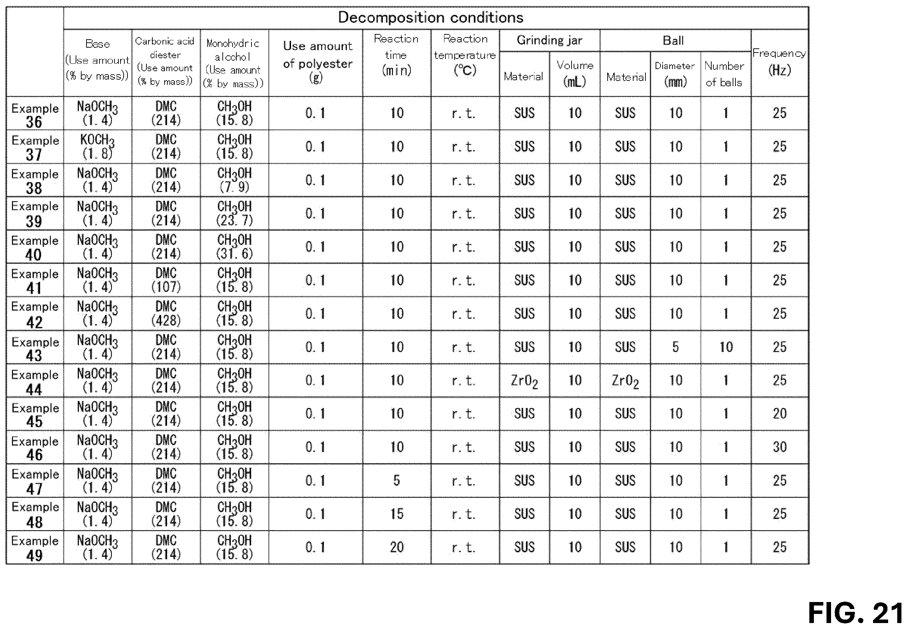

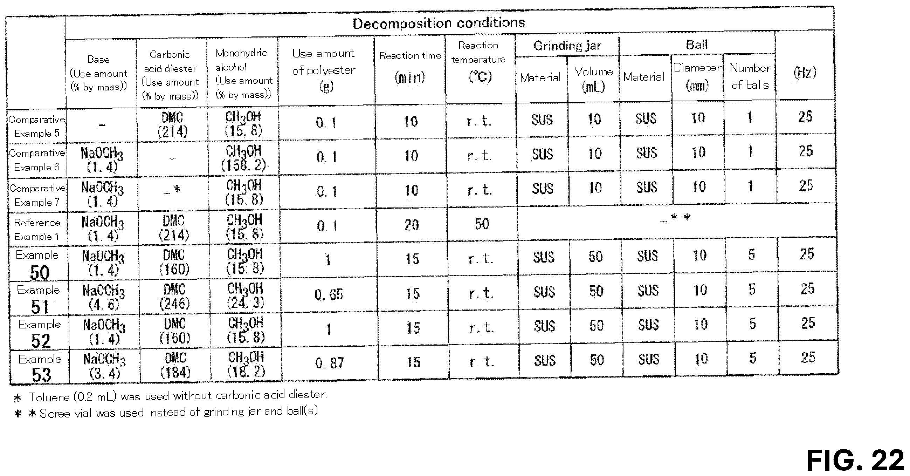

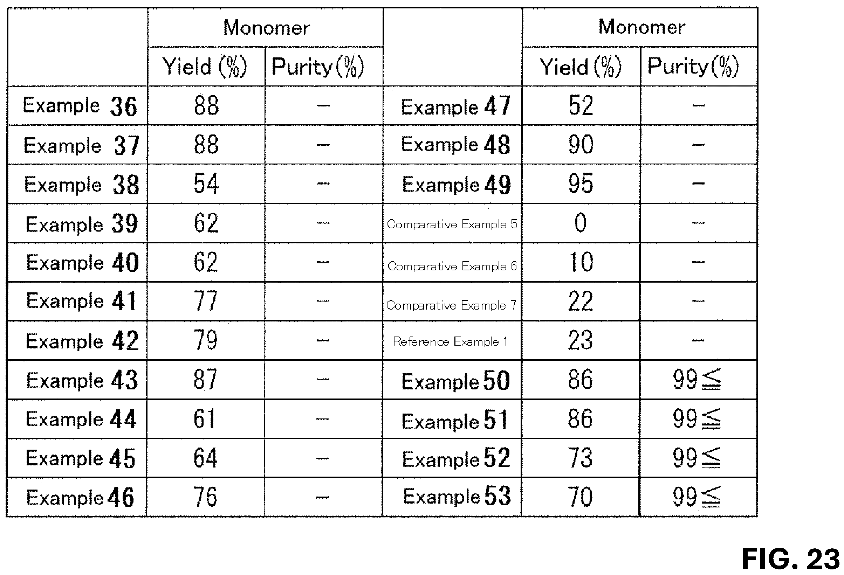

The decomposition conditions in Examples 36 to 49 are shown in Fig. 21, and the decomposition conditions in Comparative Examples 5 to 7, Reference Example 1, and Examples 50 to 53 are shown in Fig. 22. The results of Examples 36 to 53, Comparative Examples 5 to 7, and Reference Example 1 are summarized in Fig. 23.

In Fig. 21 and Fig. 22, "base (use amount (% by mass)" means "a ratio of the use amount (parts by mass) of base to the use amount (parts by mass) of polyester in the polyester-containing material". Similarly, "carbonic acid diester (use amount (% by mass)" means "a ratio of the use amount (parts by mass) of carbonic acid diester to the amount (parts by mass) of polyester in the polyester-containing material". Similarly, "monohydric alcohol (use amount (% by mass))" means "a ratio of the use amount (parts by mass) of monohydric alcohol to the amount (parts by mass) of the polyester in the polyester-containing material".

As is apparent from these figures, in Examples 36 to 49, dicarboxylic acid diesters (monomers) were obtained in good yields by a process, for the polyester in the polyester-containing material, having a low environmental load without using materials having a large environmental load such as a halogenated solvent, an organic solvent, and an excess amount of a base at room temperature.

In Examples 50 to 53, the use amount of the polyester-containing material (polyester) was increased as compared with Examples 36 to 49, and the polyester was decomposed on a larger scale. However, dicarboxylic acid diester was obtained in the same or higher yield as those in Examples 36 to 49, and moreover, the purity of the resulting dicarboxylic acid diester was high, at 99% by mass or more.

Therefore, in Examples 36 to 49, which are smaller in scale than those of Examples 50 to 53 and are more likely to cause the decomposition of polyester, it was determined that the dicarboxylic acid diesters having the same high purity as those of Examples 50 to 53 could be taken out, although the dicarboxylic acid diesters were not taken out. Furthermore, in Examples 50 to 53, such a high-purity dicarboxylic acid diester was obtained by a simple process in which the reaction solution was concentrated under reduced pressure, washed with water, and dried, without requiring additional complicated steps.

As described above, it was confirmed from Examples 36 to 53 that, according to the present embodiment, by using a ball mill, the polyester in the polyester-containing material could be efficiently decomposed into high-purity monomers in a process having a small environmental load.

On the other hand, in Comparative Examples 5 to 7 and Reference Example 1, the dicarboxylic acid diester (monomer) could not be obtained in good yield through decomposition of the polyester in the polyester-containing material, and the polyester could not be decomposed efficiently.

In Comparative Example 5, no base was used during the polyester decomposition. In Comparative Examples 6 and 7, no carbonic acid diester was used during the polyester decomposition. In Reference Example 1, no ball mill was used during the polyester decomposition.

### (Other Embodiments)

### <Polyester decomposition product recovery method>

In the embodiment described above, the recovery method of dimethyl terephthalate (produced by decomposition of PET), dimethyl 2,6-naphthalenedicarboxylate (produced by decomposition of PEN), and dimethyl 2,5-furandicarboxylate (produced by decomposition of PEF), which are ones of the polyester decomposition products, is not particularly limited. However, these substances obtained by the polyester decomposition method of the present invention can be easily isolated by distillation (including sublimation purification). As a result, dimethyl terephthalate, dimethyl 2,6-naphthalenedicarboxylate, and dimethyl 2,5-furandicarboxylate obtained by the polyester decomposition method according to the present invention can be recovered with high purity (99.0 to 99.9%).

That is, after the polyester-containing material is decomposed using the polyester decomposition method of the present invention, the solution containing the resulting polyester decomposition products can be subjected to distillation (including sublimation purification) (distillation step) without additional treatments, thereby recovering dimethyl terephthalate with high purity (99.0 to 99.9%). For distillation (including sublimation purification), a common distiller, a sublimator, or the like that is commercially available can be used.

Here, the temperature during distillation (including sublimation) is not particularly limited as long as it is 100 to 300°C, but is preferably 120 to 200°C. The pressure during distillation (including sublimation) is not particularly limited, and may be atmospheric pressure, but is preferably 5 to 5,000 Pa, and more preferably 8 to 4,000 Pa.

### [Examples 51 to 53]

The polyester decomposition product (solution) containing dimethyl terephthalate was obtained using the above-described polyester decomposition method as shown by the following three chemical reaction formulas, and then the polyester decomposition product was distilled as it is.

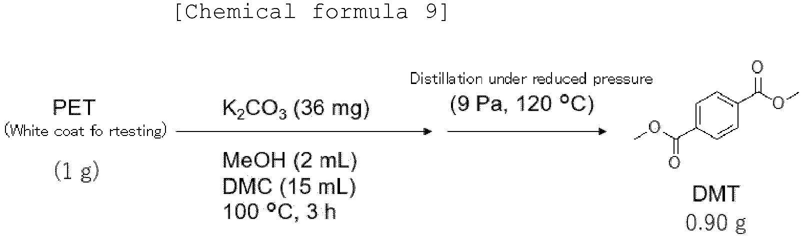

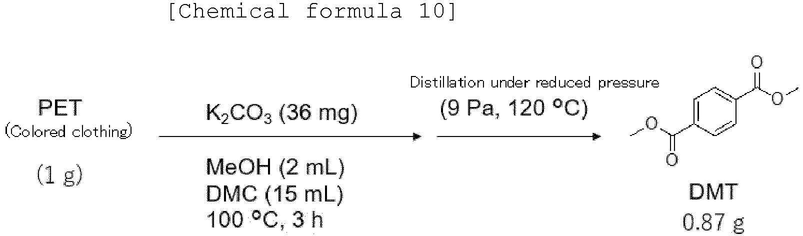

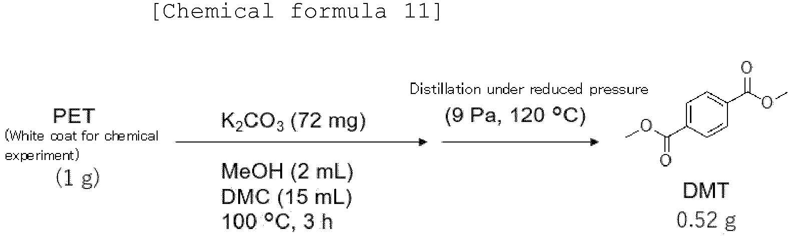

As described above, it was found that high-purity dimethyl terephthalate could be obtained by simple distillation after obtaining a polyester decomposition product (solution) containing dimethyl terephthalate using the polyester decomposition method of the present invention.

### (Incorporation by reference of basic application)

The present application claims priority based on Japanese Patent Application No. 2022-126948 filed on Aug. 9, 2023 and Japanese Patent Application No. 2022-126949 filed on Aug. 9, 2023, and the entire contents of Japanese Patent Application No. 2022-126948 and Japanese Patent Application No. 2022-126949 are incorporated herein by reference.

## Claims

1. A polyester decomposition method of a polyester contained in a polyester-containing material, the method comprising
a decomposition step of mixing a base, a monohydric alcohol, and a carbonic acid diester to decompose the polyester.

2. The polyester decomposition method according to claim 1, wherein a reaction temperature in the decomposition step is in a range of 20°C to 150°C.

3. The polyester decomposition method according to claim 1 or 2, wherein the base is an alkali metal carbonate, an alkali metal hydroxide, or an alkali metal alkoxide.

4. The polyester decomposition method according to claim 1 or 2, wherein the monohydric alcohol is methanol.

5. The polyester decomposition method according to claim 1 or 2, wherein the carbonic acid diester is dimethyl carbonate.

6. The polyester decomposition method according to claim 1 or 2, wherein the decomposition step includes mixing the base, the monohydric alcohol, and the carbonic acid diester using a mechanochemical reaction device to decompose the polyester.

7. The polyester decomposition method according to claim 6, wherein a reaction temperature in the decomposition step is in a range of 30°C to 100°C.

8. The polyester decomposition method according to claim 6, wherein the mechanochemical reaction device is a ball mill.

9. The polyester decomposition method according to claim 1 or 2, wherein the polyester-containing material contains at least one of polyester fibers and a film made of a polyester.

10. The polyester decomposition method according to claim 1 or 2, wherein the polyester-containing material contains a substance other than a polyester.

11. A polyester production method for producing a polyester using a polyester decomposition product obtained by the polyester decomposition method according to claim 1 or 2.

12. A polyester decomposition product recovery method for recovering a predetermined polyester decomposition product from a polyester decomposition product obtained by the polyester decomposition method according to claim 1 or 2, the method comprising a distillation step of distilling the polyester decomposition product, wherein
the predetermined polyester decomposition product is dimethyl terephthalate, dimethyl 2,6-naphthalenedicarboxylate, or dimethyl 2,5-furandicarboxylate.
